# EUROPÄISCHE PATENTSCHRIFT

(11) **EP 2 967 526 B1**
(45) Veröffentlichungstag und Bekanntmachung des Hinweises auf die Patenterteilung: **20.11.2019**
(21) Anmeldenummer: 14710284.2
(22) Anmeldetag: 14.03.2014
(51) Int. Cl.: A61B 17/02, A61B 17/00, A61B 90/50, A61B 90/57

(54) **CHIRURGISCHE VORRICHTUNG ZUM STABILISIEREN ODER IMMOBILISIEREN VON BEWEGTEM GEWEBE**
SURGICAL DEVICE FOR STABILIZING OR IMMOBILIZING MOVING TISSUE
DISPOSITIF CHIRURGICAL DESTINÉ À STABILISER OU À IMMOBILISER UN TISSU DÉPLACÉ

(30) Priorität: 14.03.2013 DE 102013102628
(43) Veröffentlichungstag der Anmeldung: 20.01.2016
(73) Patentinhaber: Aesculap AG, 78532 Tuttlingen (DE)
(72) Erfinder: VOGTHERR, Robert, 78532 Tuttlingen (DE); SEYFRIED, Dominik, 78126 Königsfeld (DE); ELISCH, Andreas, 78713 Schramberg (DE); BECK, Thomas, 78591 Durchhausen (DE); GENONI, Michele, 8063 Zürich (CH); WEIßHAUPT, Dieter, 78194 Immendingen (DE); MORALES, Pedro, 78532 Tuttlingen-Nendingen (DE)
(74) Vertreter: Winter, Brandl, Fürniss, Hübner, Röss, Kaiser, Polte - Partnerschaft mbB
(86) Internationale Anmeldenummer: PCT/EP2014/055172
(87) Internationale Veröffentlichungsnummer: WO 2014/140316

(56) Entgegenhaltungen:
- EP-A1- 0 808 606
- WO-A1-2011/159733
- WO-A2-01/50946
- DE-B3- 10 357 105
- GB-A- 911 419
- US-A- 3 858 578
- US-A- 5 662 300
- US-A1- 2003 216 619
- US-A1- 2008 103 491
- US-A1- 2008 214 925
- US-A1- 2012 253 116

## Beschreibung

Die vorliegende Erfindung betrifft eine chirurgische Vorrichtung zum Stabilisieren oder Immobilisieren eines Teils eines bewegten Gewebes oder auch zum Positionieren von Organen oder chirurgischen Instrumenten und Geräten während einer Operation, gemäß dem Oberbegriff des Anspruchs 1.

Es gibt unterschiedliche Arten von chirurgischen Eingriffen, bei denen der Operateur Arbeiten an bewegten Organen des menschlichen Körpers ausüben muss. Die ist beispielsweise bei einer Koronararterien-Bypass-Operation (CABG) am schlagenden Herzen der Fall. Bei einer derartigen Bypassoperation muss ein Abschnitt einer verengten Koronararterie unter Einsatz eines Transplantatgefäßes umgangen werden, wobei das Gefäß an zwei Stellen an die Arterie angenäht wird. Deshalb wird diese Operation häufig unter Verwendung einer Herz-Lungen-Maschine bei Herzstillstand durchgeführt. Sobald der Eingriff abgeschlossen ist, wird das Herz wieder zum Schlagen gebracht. Diese Operation kann auch ohne Herz-Lungen-Maschine am schlagenden Herzen durchgeführt werden. Natürlich ist diese Technik für den Operateur wesentlich schwieriger als bei einem nicht schlagenden Herzen. Deshalb benötigt man Vorrichtungen zum Stabilisieren beziehungsweise Immobilisieren von einem Teil des Herzens.

Eine Art von Stabilisierungsvorrichtungen basiert auf dem Vakuum-Prinzip. Sie weist Elemente auf, die auf einem Teil der Herzens aufsetzen, sich dort mittels Vakuum festsaugen und es somit an die Vorrichtung fixieren. Die gesamte Vorrichtung umfasst ein feststehendes Teil, welches normalerweise an einem Rippenspreizer befestigt wird, einen flexiblen Arm, bestehend aus einer Vielzahl von Einzelelementen, ein Betätigungselement, mit welchem dieser Arm gespannt wird, und ein oder mehrere Vakuum-Elemente, an welche ein Vakuumschlauch angeschlossen ist. In dem Arm befindet sich ein Zugseil, welches mittels des Betätigungselements mechanisch verspannt wird. Die Spannung des Zugseils erfolgt über ein Gewinde oder einen Exzenter. Derartige Vorrichtungen sind beispielsweise aus den Patentschriften US 6,866,628 B2, US 7,311,664 B2, US 7,399,272 B2, US 7,476,196 B2 oder US 7,479,104 B2 bekannt.

Sehr ähnlich ist die zweite Art von Stabilisierungsvorrichtungen, die ohne Vakuum arbeitet. Hier besteht die Technik mit Ausnahme der Vakuum-Elemente aus denselben Bauteilen. Anstatt dieser Elemente gibt es hier funktionslose, inaktive, insbesondere gabelförmige, Stabilisierungselemente, die auf einen Teil des Herzens gedrückt werden, um es somit rein mechanisch zu stabilisieren. Diese Technik wird zum Beispiel in US 6,581,889B2 bzw. WO 01/50946 A2 beschrieben.

Medizinische Stabilisierungsvorrichtungen werden des Weiteren auch in WO 2011/159733 A1, US 3,858,578 A, US 2003/0216619 A1, US 2008/0214925 A1 und US 911,419 A1 offenbart. In EP 0 808 606 A1 wird ein artverwandter Retraktor beschrieben.

Medizinische Systeme, die modular aufgebaut sind, werden beispielsweise in US 5,662,300 A, DE 103 57 105 B3, US 2008/0103491 A1 und US 2012/0253116 A1 offenbart.

Nachteilig an den bisherigen Lösungen ist, dass der Anwender bei allen üblichen Vorrichtungen für die Handhabung dieser Art mit beiden Händen arbeiten muss. Am proximalen Ende arretiert er dabei den flexiblen Arm mittels des dafür vorgesehenen Betätigungselements, während er das distale Ende führt und auf das Gewebe drückt. Der flexible Gliederarm, der an seinem proximalen Ende noch eine Drehachse besitzt, mittels welchem er in horizontaler Ebene zum feststehenden Teil, das auf dem Spreizer befestigt ist, verschwenkt werden kann, verkompliziert diesen Vorgang nochmals. Diese Drehachse wird in vielen Fällen mittels desselben Zugseils gleichzeitig mit dem Arm arretiert. Im Extremfall benötigt der Anwender dann auch noch einen Assistenten, der ihm zum Beispiel diese Drehachse während des Arretierungsvorganges in der gewünschten Position hält.

Darüber hinaus ist bei diesen Vorrichtungen oftmals nicht klar, wie stark das Zugseil gespannt ist. Verschiedene Anwender werden, abhängig von eigenem Gefühl und Kraft, das Betätigungselement, welches oftmals als Schraubmechanismus ausgeführt ist, verschieden stark betätigen und damit sowohl unterschiedliche Zugkräfte als auch unterschiedliche Steifigkeiten des Gliederarmes erzeugen.

Ein weiterer Nachteil ist die finanziell hohe Aufwendung für eine solche Vorrichtung. Dadurch, dass die einzelnen Armsegmente und das Zugseil, welches hinsichtlich Flexibilität aus mehreren Einzellitzen besteht, kaum effektiv reinigbar sind, ist das gesamte Instrument nicht wiederverwendbar und wird nur ein einziges Mal eingesetzt.

Aufgabe der vorliegenden Erfindung ist die Bereitstellung einer chirurgische Vorrichtung zum Stabilisieren oder Immobilisieren von sich bewegenden Organen, welche nicht unter den oben geschilderten Nachteilen leidet. Insbesondere soll eine Vorrichtung bereitgestellt werden, welche dem Operateur die Handhabung erleichtert, insbesondere eine Einhandbedienung. Ferner sollte die Bedienbarkeit der Vorrichtung personenunabhängig sein. Schließlich sollten die laufenden Kosten einer solchen Vorrichtung verringert werden.

Diese Aufgabe wird durch die Merkmale des Anspruchs 1 gelöst.

Eine erfindungsgemäße chirurgische Vorrichtung, mit welcher bewegtes Gewebe stabilisiert, immobilisiert oder gehalten werden kann oder Organe positioniert werden können, z.B. ein Teil eines schlagenden Herzens bei einer Koronararterien-Bypass-Operation, weist einen Grundkörper auf, an dem ein flexibler Arm, z.B. in Form eines Gliederarms, befestigt ist oder befestigbar ist. Dieser Gliederarm kann in unterschiedliche Positionen und/oder Lagen gebracht werden. An seinem freien Ende ist zumindest ein Halteelement angeordnet, mit welchem das entsprechende Gewebe oder Organ gehalten bzw. stabilisiert werden kann. Über einen Spannmechanismus lässt sich Gliederarm in einer gewünschten Stellung feststellen. Erfindungsgemäß erfolgt das Spannen und/oder Lösen des Spannmechanismus bzw. das Feststellen und Lösen des Gliederarms mittels einer Energiequelle.

Das Spannen und/oder Lösen des flexiblen Arms geschieht somit nicht wie bei den meisten der oben genannten Vorrichtungen rein mechanisch durch die Aufbringung von Handkraft, sondern wird von einer beispielsweise hydraulischen, pneumatischen oder elektrischen, Energiequelle übernommen oder zumindest unterstützt. Dies ermöglicht eine Einhandbedienung der Vorrichtung, da der Chirurg den Spannmechanismus nicht selbst mit den Händen spannen und/oder lösen muss, wozu er in der Regel zwei Hände benötigt, nämlich eine zum Halten der Vorrichtung und die andere zum Spannen bzw. Lösen. Durch die Einhandbedienung ist der flexible Arm einfach positionierbar und arretierbar und unterstützt den Operateur z.B. bei der Stabilisierung von Gewebe oder der Positionierung von Organen.

Ein weiterer Vorteil einer Energie- oder Kraftquelle ist darin zu sehen, dass die Spann- bzw. Lösekraft genauer eingestellt werden kann und das Spannen des Spannmechanismus stets mit der gleichen Spannkraft erfolgt und deshalb nicht in Abhängigkeit von der jeweiligen Bedienperson variiert. Somit kann sichergestellt werden, dass der festgestellte bzw. arretierte Arm stets die gewünschte optimale Steifigkeit aufweist und Haltekraft bereitstellt und nicht je nach Anwender zu schwach oder zu stark gespannt wird. Ersteres könnte dazu führen, dass der Arm während der Operation nachgibt oder das zu haltende Gewebe oder Organ freigibt, und Letzteres könnte zu einem Überspannen und Zerstören der Spannmechanik und somit zur Unbrauchbarkeit der Vorrichtung führen.

Der Gliederarm bietet eine Vielzahl weiterer Anwendungsmöglichkeiten, wie beispielsweise zum Halten von Wundhaken oder Spateln im Bereich der Neurochirurgie oder als flexibler Haltearm zum Halten und Positionieren weiteren Equipments wie beispielsweise eines Trokars oder einer Kamera zur Durchführung oder Unterstützung eines chirurgischen Eingriffs. Das Halteelement des Gliederarms kann dafür mit einem universellen Adapter versehen sein, um den Anschluss verschiedenster einmal- oder wiederverwendbarer chirurgischer Instrumente und Geräte, die mittels des Gliederarms während eines chirurgischen Eingriffs gehalten und positioniert werden, zu ermöglichen.

Gemäß der Erfindung erfolgt das Spannen und das Lösen des Spannmechanismus bzw. das Feststellen und Lösen des Gliederarms auf unterschiedliche Weise. Das Spannen des Spannmechanismus erfolgt selbsttätig oder automatisch, d.h. ohne irgendwelches Zutun des Anwenders, und das Lösen des Spannmechanismus erfolgt durch Ansteuern oder Aktivieren der Energiequelle. So erfolgt das Spannen des Spannmechanismus mittels Federkraft, so dass der Spannmechanismus den Gliederarm in der jeweiligen Position spannt und feststellt und der Anwender den Gliederarm lösen und flexibel machen kann, indem er die Energiequelle entsprechend ansteuert. Der Vorteil der Federvorspannung liegt insbesondere darin, dass durch die Federkraft über einen gewissen Weg eine quasi kontinuierliche Kraft zur Verfügung gestellt wird und diese Kraft bei jeder Anwendung für die gleiche, optimale Vorspannung des Spannmechanismus und für die optimale Steifigkeit des Arms sorgt. Ein weiterer Vorteil der selbsttätigen Vorspannung des Spannmechanismus, wie z. B. mit einer Feder, liegt darin, dass der Spannmechanismus lediglich zum Lösen vom Anwender bedient werden muss und, wenn der Anwender die Vorrichtung bzw. das entsprechende Betätigungselement für die Ansteuerung der Energiequelle loslässt, selbsttätig in seiner arretierten Lage verbleibt.

Gemäß der Erfindung weist der Spannmechanismus ein oder mehrere Zugseile auf, die durch eine Vielzahl von zueinander beweglichen, insbesondere komplementär ausgebildeten Gliederelementen des Gliederarms geführt sind und über welche die Gliederelemente reibschlüssig gegeneinander verspannt werden können. Über die Energiequelle lässt sich eine entsprechende Stellmechanik bzw. Zylinder-Kolben-Mechanik betätigen, welche mit dem Zugseil in Verbindung steht und dieses entweder spannt oder freigibt.

Die erfindungsgemäße Stellmechanik bzw. Zylinder-Kolben-Mechanik wandelt die von der Energiequelle zugeführte Energie in eine entsprechende mechanische Kraft um, um das Zugseil zu betätigen.

Gemäß der Erfindung ist die Vorrichtung modular aufgebaut, wobei sich der Gliederarm und der Grundkörper mechanisch lösbar miteinander verbinden lassen, so dass der Gliederarm aufgrund der Reinigungsschwierigkeiten der einzelnen Gliederelemente und des Zugseils als auswechselbares Einwegmodul verwendet werden kann und der Grundkörper mit der Stellmechanik bzw. der Zylinder-Kolben-Mechanik und Steuerungselementen der Energiequelle als wieder verwendbarer Technikblock vorgesehen ist. Die Schnittstelle vom Gliederarm und Grundkörper ist so konfiguriert, dass neben der mechanischen Kopplung auch eine funktionale Kopplung zwischen dem Zugseil und einem Stellorgan bzw. einem Schieber des Spannmechanismus erfolgt und gegebenenfalls weitere Schnittstellen, wie Steuerleitungen, elektrische Kontakte, etc. verbunden werden. Durch wieder verwendbare Komponenten, d.h. dem Technikblock, entsteht eine Kostenersparnis zu vergleichbaren Produkten. Ein wesentlicher weiterer Vorteil des modularen Aufbaus ist darin zu sehen, dass der Technikblock je nach Anwendung mit verschiedenen Arbeitsenden bzw. Gliederarmen mit unterschiedlichen Größen oder unterschiedlichen Effektoren oder Halteelementen kombinierbar ist.

Vorteilhafte Weiterbildungen sind Gegenstand der Unteransprüche.

Bei der Energiequelle kann es sich um eine externe Energiequelle handeln, die sich außerhalb der chirurgischen Vorrichtung befindet. Dies ist von Vorteil, wenn man die chirurgische Vorrichtung und insbesondere den Grundkörper raumsparend konstruieren will. Die externe Anordnung der Energiequelle kann auch dann besonders vorteilhaft sein, wenn als Energiequelle ein austauschbarer Energiespeicher vorgesehen ist. Durch die externe Anordnung wird dann nämlich das Austauschen eines aufgebrauchten Energiespeichers besonders erleichtert.

Die Energiequelle, welche zum Spannen und/oder Lösen des Spannmechanismus verwendet wird, kann über ein oder mehrere manuell betätigbare Betätigungselemente ansteuerbar bzw. aktivierbar sein. Wenn sich das Betätigungselement auf der distalen Seite des Gliederarms, d.h. in der Nähe des Halteelements befindet, hat dies den Vorteil, dass der Chirurg mit einer Hand das Halteelement an dem entsprechenden Gewebe oder Organ positionieren kann und gleichzeitig über das Betätigungselement in der gewünschten Lage feststellen kann bzw. umgekehrt nach erfolgtem Eingriff lösen und von dem Gewebe bzw. Organ entfernen kann. Bei den eingangs genannten Vorrichtungen aus dem Stand der Technik erfolgt die Feststellung des Gliederarms über einen am proximalen Ende des Gliederarms angeordneten Drehknopfs, der entsprechend weit von der eigentlichen Operationsstelle entfernt ist. Jedoch ist es von Vorteil, wenn die Energiequelle neben dem distalen Betätigungselement auch noch über ein auf der proximalen Seite des Gliederarms angeordnetes Betätigungselement angesteuert werden kann. Wenn nämlich das Halteelement derart tief in der Körperhöhle des Patienten versenkt ist, dass das distale Betätigungselement nicht oder nur schlecht erreichbar ist, kann der Anwender mit dem Betätigungselement auf der proximalen Seiten des Gliederarms, der sich außerhalb des Patienten befindet, den Arm lösen und gegebenenfalls neu positionieren. Dies ermöglicht einen flexiblen Einsatz der chirurgischen Vorrichtung. Alternativ kann das Betätigungselement auf dem Grundkörper ebenso das einzige Betätigungselement darstellen.

Die Energiequelle kann von beliebiger Art sein, solange sie eine Energie liefert, die zum Betätigen des Zugmechanismus in zumindest eine Richtung geeignet und ausreichend ist. Die Energiequelle kann beispielsweise eine hydraulische oder pneumatische Druckquelle sein, die Stellmechanik bzw. Zylinder-Kolben-Mechanik kann eine hydraulisch oder pneumatisch betätigbare Zylinder-Kolben-Mechanik sein und über das zumindest eine Betätigungselement kann ein Fluidsteuerungselement, z. B. ein Ventil oder eine Ventilanordnung, zum Steuern des an der Zylinder-Kolben-Mechanik wirkenden Fluiddrucks angesteuert werden. Auch kann die Energiequelle ein Speicher für elektrische Energie, insbesondere eine Batterie oder ein Akkumulator sein, die Stellmechanik bzw. Zylinder-Kolben-Mechanik kann eine elektromotorisch betätigbare Zylinder-Kolben-Mechanik sein und über das zumindest eine Betätigungselement kann ein elektrisches oder elektronisches Steuergerät zur Steuerung eines Elektromotors, der die Zylinder-Kolben-Mechanik verstellen kann, angesteuert werden. Der Elektromotor befindet sich vorzugsweise wie die Zylinder-Kolben-Mechanik im Grundkörper der chirurgischen Vorrichtung.

Gemäß einem Aspekt der Erfindung wird das Zugseil des Zugmechanismus über ein an der Zylinder-Kolben-Mechanik wirkende Feder vorgespannt und über eine an der Zylinder-Kolben-Mechanik wirkende Druckluft von einer Druckluftquelle, insbesondere von einer Druckluftkartusche oder einem standardisierten Druckluftanschluss, entspannt. Die Verwendung einer Druckluftkartusche hat den Vorteil, dass sie ein völlig autonomes Arbeiten ermöglicht. Alternativ kann die Zylinder-Kolben-Mechanik über einen standardisierten Druckluftanschluss und einem Schlauch oder einer Leitung mit der Luftversorgung, die sich in jedem Operationssaal befindet, verbunden sein.

Somit kann je nach Anwendungsfall die chirurgische Vorrichtung mit einer Druckgaskartusche oder einer vorhandenen Druckluftquelle verbunden werden.

Gemäß einem zusätzlichen oder alternativen Aspekt der Erfindung können das Steuermedium, das zum Steuern des Fluidsteuerungselements verwendet wird, und das Arbeitsmedium, das zum Betätigen der Zylinder-Kolben-Mechanik verwendet wird, unterschiedlich sein. So kann beispielsweise der Kolben der Zylinder-Kolben-Mechanik mit Druckluft betätigt werden, während die Steuerung des Ventils, welches die Druckluft auf den Druckluftkolben wirken lässt oder nicht, elektrisch angesteuert werden. Die dafür benötigten Leitungen lassen sich wesentlich platzsparender zwischen dem Betätigungselement und dem Fluidsteuerungselement unterbringen. Alternativ kann auch dasselbe Medium als Steuer- und Arbeitsmedium verwendet werden, wobei bei der Verwendung von Druckluft für das Steuermedium zum Ansteuern des Ventils ein wesentlich geringerer Druck als für das Arbeitsmedium zum Betätigen des Druckluftkolbens verwendet werden kann und somit die Steuerleitungen wesentlich dünnwandiger und flexibler ausgeführt sein können und das Betätigungselement insgesamt kompakter gestaltet werden kann. In anderen Ausführungen kann auch eine Energiequelle ausreichend sein.

Die Zylinder-Kolben-Mechanik, die sich vorzugsweise im Grundkörper der Vorrichtung befindet, ist über entsprechende Anschlüsse und Leitungen mit den externen Steuerenergiequellen und Arbeitsenergiequellen verbunden.

Gemäß einem alternativen Aspekt der Erfindung wird das Zugseil des Zugmechanismus über eine an der Zylinder-Kolben-Mechanik wirkende Feder vorgespannt und durch eine vom Elektromotor hervorgerufene und an der Zylinder-Kolben-Mechanik wirkende Kraft entspannt. Der Elektromotor, der insbesondere auch als Linearmotor ausgebildet sein kann, bezieht seine Energie von einer elektrischen Energiequelle, insbesondere einem Stromanschluss oder alternativ oder zusätzlich von einem Energiespeicher wie einer Batterie oder einem Akkumulator. Die Verwendung eines Energiespeichers hat den Vorteil, dass er ein völlig autonomes Arbeiten ermöglicht. Alternativ kann der Elektromotor für die Zylinder-Kolben-Mechanik über einen standardisierten Stromanschluss und einer Leitung mit der Stromversorgung, die sich in jedem Operationssaal befindet, verbunden sein.

Der Grundkörper der Vorrichtung kann über einen Befestigungsabschnitt an einer im oder am Operationsfeld befindlichen Haltevorrichtung, insbesondere einem Sternumspreizer, befestigbar sein. Wenn das Halteelement am Gewebe bzw. Organ positioniert ist und der flexible Arm über den Spannmechanismus festgestellt ist, wird das Gewebe bzw. Organ über das Halteelement, dem festgestellten Arm, dem Grundkörper und der ortsfesten Haltevorrichtung in Position gehalten.

Gemäß einem Aspekt der Erfindung kann der Gliederarm zum Grundkörper drehbar gelagert sein und das Halteelement zum Gliederarm schwenkbar gelagert sein. Da der Gliederarm darüber hinaus in allen Richtungen biegbar ist, lässt sich das Halteelement aufgrund der diversen Freiheitsgrade in jede gewünschte Position bringen. Vorzugsweise wird die Schwenkbarkeit des Halteelements bezüglich des Gliederarms und die Drehbarkeit des Gliederarms bezüglich des Grundkörpers unterbunden bzw. beschränkt, wenn der Spannmechanismus gespannt wird, so dass nicht nur die Form des Gliederarms, sondern auch die Position des Halteelements zum Gliederarm bzw. die Position zum Grundkörper festgelegt werden.

Wenn sich das Betätigungselement zum Ansteuern der Energiequelle auf der distalen Seite des Gliederarms befindet und das Fluidsteuerungselement, das elektrische oder elektronische Steuergerät bzw. das Stellorgan für die Stellmechanik bzw. die Zylinder-Kolben-Mechanik des Spannmechanismus auf der distalen Seite des Gliederarms befindet, insbesondere im Grundkörper vorgesehen ist, muss eine Steuerleitung vom Betätigungselement zu dem Fluidsteuerungselement, dem elektrischen oder elektronischen Steuergerät bzw. Stellorgan führen. Diese Signal- oder Energieübertragungsleitungen können sich im oder außerhalb des Gliederarms befinden. Vorteilhafterweise verlaufen diese jedoch durch das Innere des Gliederarms und sind somit vor äußeren Einflüssen geschützt. Um die Leitungen durch das Innere des Gliederarms zu führen, können die jeweiligen Gliederelemente des Gliederarms jeweils eine zentrale Zugseildurchführung für das Zugseil und zumindest eine außermittig angeordnete Aussparung für Leitungen, insbesondere für eine Steuerleitung zwischen dem distalen Betätigungselement und dem Fluidsteuerungselement bzw. dem elektrischen oder elektronischen Steuergerät, aufweisen. Durch die separat ausgebildeten Aussparungen für die Leitungen wird sichergestellt, dass die Leitungen nicht mit dem Zugseil des Spannmechanismus in Kontakt kommen bzw. sich gegenseitig beeinflussen. Anstatt die Zugseildurchführung und die Leitungsaussparung in den Gliederelementen vorzusehen, kann alternativ jedes der Gliederelemente mit einer separaten zentral und drehbar gelagerten Drehscheibe versehen sein, in welcher die Zugseildurchführung und die Leitungsaussparung vorgesehen sind. Durch die Relativdrehung dieser Drehscheibe bezüglich der Gliederelemente kann eine Verdrehung zwischen den Gliederelementen, welche andernfalls zu einer Verletzung der Leitungen führen könnte, ausgeglichen werden. Alternativ können die Gliederelemente mit einer Drehsicherung, insbesondere mit ineinandergreifenden Sperrelementen, versehen sein, um eine Verdrehung der Gliederelemente um das Zugseil zu beschränken. Auch durch diese Variante wird einer Verletzung der Steuerleitungen vorgebeugt.

Gemäß einem Aspekt der Erfindung nimmt die Größe der Gliederelemente mit zunehmendem Abstand von dem Grundkörper und in Abhängigkeit des zu erwartenden Biegemomentenverlaufs am Gliederarm ab, um so eine einheitliche Biegung des Arms zu erreichen.

Gemäß einem alternativen Aspekt der Erfindung nimmt die Größe von Kegelwinkeln von kegelförmigen Kontaktinnenflächen der Gliederelemente mit zunehmendem Abstand von dem Grundkörper und in Abhängigkeit des zu erwartenden Biegemomentenverlaufs am Gliederarm zu, um so eine einheitliche Biegung des Arms zu erreichen. Die Außenabmaße der Gliederelemente können so über die gesamte Länge des Gliederarms konstant gehalten werden.

Gemäß einem Aspekt der Erfindung befindet sich die Energiequelle bzw. der Energiespeicher bei der modular aufgebauten Vorrichtung außerhalb des Grundkörpers. Insbesondere kann die Energiequelle bzw. der Energiespeicher in den Gliederarm einsetzbar oder an diesen anschließbar sein. Alternativ kann der Energiespeicher fest in den Gliederarm integriert und zur Einmalverwendung konzipiert sein.

Gemäß einem Aspekt der Erfindung können die Energiequelle in Form eines autarken Energiespeichers und der demontierbare Arm so ausgeformt sein, dass sie miteinander verbindbar sind. Eine solche Ausführung ist dann vorteilhaft, wenn die jeweiligen für den Grundkörper vorgesehenen Anschlüsse des Arms und des Energiespeichers so ausgerichtet sind, dass ein Verbund aus Arm und Energiespeicher mit einem einzigen Handgriff bzw. mit einer einzigen Handhabung am Grundkörper angebracht bzw. angeschlossen werden kann.

Wird die Verbindung zwischen Arm und Energiespeicher dabei fest ausgebildet und ist die vom Energiespeicher gespeicherte Energie nur nutzbar zwischen erstmaliger Montage des festen Arm-Energiespeicher-Verbunds an dem Grundkörper und erstmaliger Demontage des festen Arm-Energiespeicher-Verbunds von dem Grundkörper, ergibt sich daraus ein weiterer vorteilhafter Effekt in Hinblick auf die Sicherheit. Fest bedeutet in diesem Zusammenhang nicht oder nur aufwendig, insbesondere nicht werkzeuglos, lösbar. Soll der demontierbare Arm aus hygienischen Gründen nicht mehrmals verwendet werden ist es vorteilhaft, den Arm überhaupt nur für die einmalige Benutzung verwendbar machen. Dies wird gemäß diesem Aspekt der Erfindung dadurch gewährleistet, dass der Energiespeicher nur nach dem ersten Andocken an den Grundkörper Energie an den Grundkörper abgeben kann, und dass die feste Verbindung zwischen dem Energiespeicher und dem Arm mit dem Wechsel des Energiespeichers auch den Wechsel des Arms erfordert. Dass die Energie nur zwischen dem erstmaligen Andocken und dem erstmaligen Abdocken des Energiespeichers nutzbar ist, wird, wenn es sich bei dem Energiespeicher um eine Druckkartusche handelt, mittels einer versiegelnden Membran, und, wenn es sich bei dem Energiespeicher um einen elektrischen Energiespeicher handelt, mittels eines Entlademechanismus bewerkstelligt.

Wenn die anzuschließende Energiequelle, wie z. B. eine Druckgaskartusche, eine Batterie, ein Akkumulator, ein Schlauch oder eine Leitung unsteril sind und deshalb nicht unmittelbar an dem Grundkörper der Vorrichtung, die sich in unmittelbarer Nähe des Operationsfeldes befindet, angeschlossen werden können, ist gemäß einem anderen Aspekt der vorliegenden Erfindung zwischen Grundkörper und Energiequelle eine Adaptionseinheit zwischengeschaltet, welche die Trennstelle zwischen sterilem und insterilem Bereich bildet und welche einerseits über lösbare Leitungen mit dem Grundkörper und andererseits mit der Energiequelle verbunden ist.

Gemäß einem Aspekt der Erfindung können sich Teile der Stell- oder Steuerorgane, insbesondere solche, die mehr Bauraum beanspruchen als der Grundkörper bei kompakter Bauweise bereitstellen, in der Adaptionseinheit vorgesehen sein.

Ein weiterer Aspekt der vorliegenden Erfindung betrifft einen chirurgischen Arbeitsarm zum Stabilisieren oder Immobilisieren von bewegtem Gewebe oder zum Positionieren von Organen, insbesondere eines Teils eines schlagenden Herzens, der insbesondere in einer erfindungsgemäßen chirurgischen Vorrichtung verwendet werden kann. Ein solcher Arbeitsarm weist einen in unterschiedliche Positionen und/oder Lagen bringbaren, flexiblen Gliederarm mit einer Vielzahl von aneinander gereihten, zueinander beweglichen, insbesondere komplementär ausgebildeten, Gliederelementen und zumindest einen am distalen Ende des Gliederarms angeordnetes Halteelement auf. Erfindungsgemäß ist ferner ein Kopplungsabschnitt zur mechanischen und funktionalen Anbindung des Arbeitsarms an einer chirurgischen Vorrichtung vorgesehen, wobei ein Zugseil, das durch die Gliederelemente geführt ist und über welches die Gliederelemente reibschlüssig gegeneinander verspannt werden können, einen proximalen Verbindungsabschnitt aufweist, der mit einem in der chirurgischen Vorrichtung bzw. einem Technikblock einer chirurgischen Vorrichtung vorgesehenen Spannmechanismus, insbesondere formschlüssig, werkzeuglos verbindbar ist.

Der Vorteil dieses Arbeitsarms bzw. Arbeitsarmmoduls liegt insbesondere darin, dass dieser kostengünstig herstellbar ist und keinerlei kostenintensive Steuerorgane oder -mimiken für den Spannmechanismus aufweist, sondern lediglich Schnittstellen zur Anbindung an diese bereitstellt. Somit kann dieser Arbeitsarm zur Einmalverwendung vorgesehen werden. Ferner können unterschiedliche Typen eines solchen Arbeitsarms zur Verwendung mit dem gleichen Technikblock vorgesehen werden, solange ein identischer oder standardisierter Kopplungsabschnitt verwendet wird.

Der chirurgische Arbeitsarm kann ferner ein am distalen Ende des Gliederarms vorgesehenes Betätigungselement zur Ansteuerung des in der chirurgischen Vorrichtung vorgesehenen Spannmechanismus und Steuerleitungen aufweisen, welche das Betätigungselement mit Schnittstellen verbindet, die im Kopplungsabschnitt zur Übertragung der Ansteuerungssignale oder -befehle zur chirurgischen Vorrichtung vorgesehen sind. Die Steuerleitungen können außerhalb, vorzugsweise jedoch innerhalb des Gliederarms verlaufen.

Weitere Vorteile und Ausgestaltungen der erfindungsgemäßen Vorrichtung ergeben sich aus der folgenden Beschreibung einer bevorzugten Ausführungsform der Erfindung.

### Kurze Beschreibung der Zeichnungen

Fig. 1 zeigt eine perspektivische Ansicht einer chirurgischen Vorrichtung gemäß einer ersten Ausführungsform der Erfindung;
Fig. 2 zeigt einen Technikblock mit gekoppeltem Gliederarm der in der Fig. 1 gezeigten chirurgischen Vorrichtung;
Fig. 3 zeigt eine perspektivische Ansicht des Technikblocks;
Fig. 4 zeigt eine Querschnittsansicht des in der Fig. 3 gezeigten Technikblocks;
Fig. 5 zeigt eine perspektivische Ansicht des Gliederarms;
Fig. 6 zeigt eine vergrößerte Ansicht eines proximalen Abschnitts des Gliederarms;
Fig. 7 zeigt eine vergrößerte Teilansicht des proximalen Abschnitts des Gliederarms;
Fig. 8 zeigt eine perspektivische Ansicht eines einzelnen Gliederelements gemäß einer ersten Ausführungsform der Erfindung;
Fig. 9 zeigt eine perspektivische Ansicht eines einzelnen Gliederelements gemäß einer zweiten Ausführungsform der Erfindung;
Fig. 10 zeigt eine perspektivische Ansicht von zwei Gliederelementen gemäß einer dritten Ausführungsform der Erfindung;
Fig. 11 zeigt eine perspektivische Ansicht einer chirurgischen Vorrichtung gemäß einer nicht beanspruchten vierten Ausführungsform;
Fig. 12a zeigt eine vordere perspektivische Ansicht des Gliederarms der chirurgischen Vorrichtung gemäß der vierten Ausführungsform;
Fig. 12b zeigt ein Detail der Fig. 12a;
Fig. 13a zeigt eine rückwärtige perspektivische Ansicht des Gliederarms der chirurgischen Vorrichtung gemäß der vierten Ausführungsform;
Fig. 13b zeigt ein Detail der Fig. 13a;
Fig. 14 zeigt eine vordere perspektivische Ansicht des Technikblocks der chirurgischen Vorrichtung gemäß der vierten Ausführungsform; und
Fig. 15 zeigt eine Querschnittsansicht des in der Fig. 14 gezeigten Technikblocks.

### Detaillierte Beschreibung bevorzugter Ausführungsformen

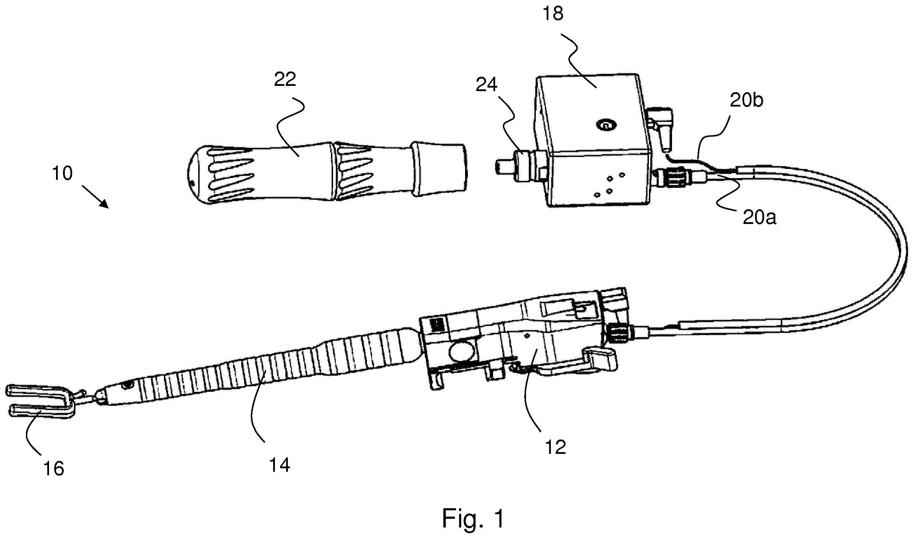

Fig. 1 zeigt eine perspektivische Ansicht einer chirurgischen Vorrichtung 10 zum Stabilisieren oder Immobilisieren eines Teils eines bewegten Gewebes oder auch zum Positionieren von Organen gemäß einer ersten Ausführungsform der Erfindung. Die Vorrichtung 10 weist einen Grundkörper oder Technikblock 12 auf, an dem ein flexibler Gliederarm 14 befestigt ist, an dessen freien Ende ein Halteelement 16 zum Halten des Gewebes bzw. Organs vorgesehen ist. Die Vorrichtung 10 weist ferner eine Adaptionseinheit 18 auf, welche über mehrere Leitungen 20 mit dem Technikblock 12 in Verbindung steht. Eine Druckluftkartusche 22, die als externe Energiequelle dient und die das zum Betätigen des im Technikblock 12 integrierten Spannmechanismus für den Gliederarm 14 notwendige Arbeitsmedium liefert, kann an einem Druckluftanschluss 24 an der Adaptionseinheit 18 angeschlossen werden. Über eine Druckluftleitung 20a wird die Druckluft von der Adaptionseinheit 18 an den Technikblock 12 übertragen. Eine parallel dazu verlaufende Signalleitung 20b dient zur Steuerung der in der Adaptionseinheit 18 vorgesehenen (nicht gezeigten) Fluidsteuerelemente bzw. Ventile, welche vom Technikblock 12 bzw. vom Gliederarm 14 angesteuert werden können.

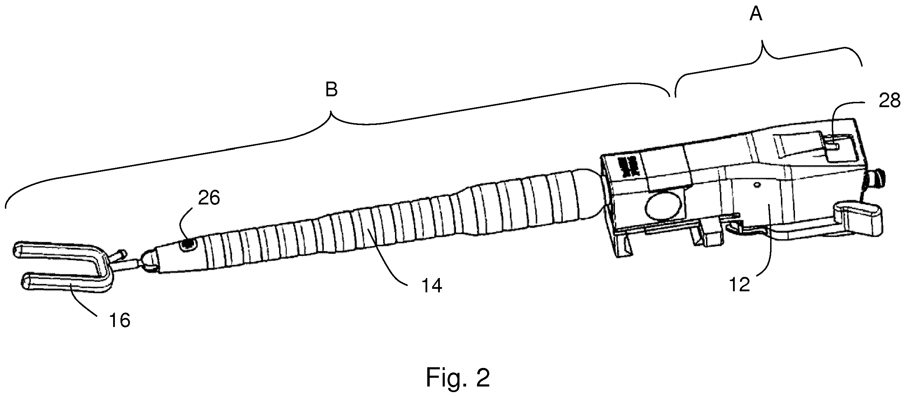

Die Fig. 2 zeigt lediglich den Technikblock 12, den Gliederarm 14 und das Halteelement 16 in einer vergrößerten Darstellung. Der Technikblock 12 enthält im Wesentlichen die gesamte Technik zum Spannen des Gliederarms 14 sowie zum Fixieren des Gesamtsystems an nicht gezeigten externen Halterungen, wie z. B. einen Sternumspreizer. Dabei bildet der Technikblock 12 ein wiederverwendbares Technikmodul A und der Gliederarm 14 samt Halteelement 16 ein für die Einmalverwendung konzipiertes Arbeitsmodul B.

Aus der Fig. 2 ist ferner erkennbar, dass am distalen Ende des Gliederarms 14 ein erstes Betätigungselement 26 in Form eines elektrischen Tasters und am Technikblock 12 ein zweites Betätigungselement 28, ebenfalls in Form eines elektrischen Tasters, vorgesehen sind. Beide Betätigungselemente 26 und 28 dienen zur Steuerung des Energieflusses von der externen Energiequelle, d.h. zur Steuerung der Druckluft, die von der Druckluftkartusche 22 über die Druckluftleitung 20a und einen Druckluftanschluss 30 zum Technikblock 12 gelangt. Die Betätigungselemente 26, 28 bzw. die Ansteuerung der externen Energiequelle können so ausgestaltet sein, dass der Energiefluss solange freigegeben wird, solange die Betätigungselemente 26, 28 gedrückt werden. Alternativ kann durch einmaliges Betätigen der Energiefluss freigegeben werden und durch nochmaliges Betätigen wieder unterbunden werden.

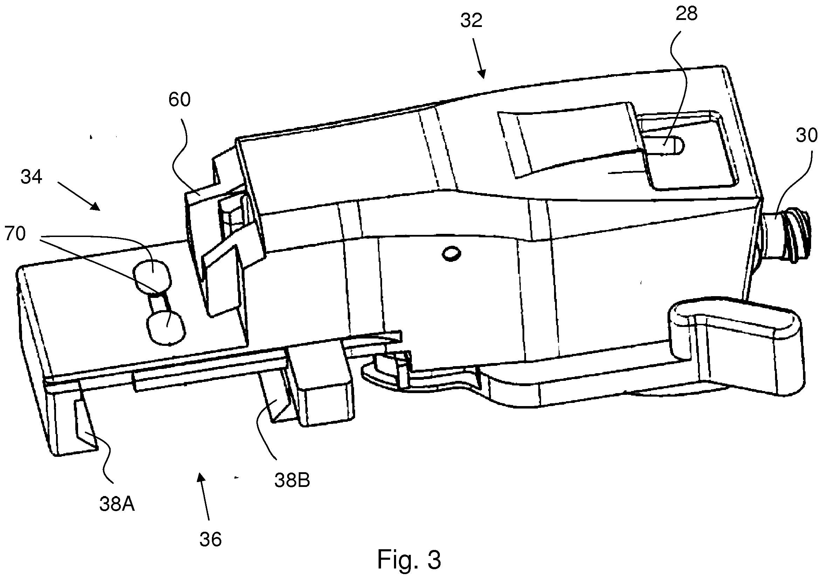

Die Fig. 3 zeigt eine perspektivische Ansicht des Technikblocks 12 alleine. Der Technikblock 12 weist einen Gehäuseabschnitt 32, einen Kopplungsabschnitt 34 zum Ankoppeln des Arbeitsmoduls B und einen Befestigungsabschnitt 36 zum Befestigen des Technikblocks 12 an einer nicht gezeigten Halterung, wie einem Sternumspreizer. Der Gehäuseabschnitt 32 kann mit ergonomischen Griffmulden versehen werden, so dass die Montage bzw. Demontage des Arbeitsmoduls B vereinfacht werden kann.

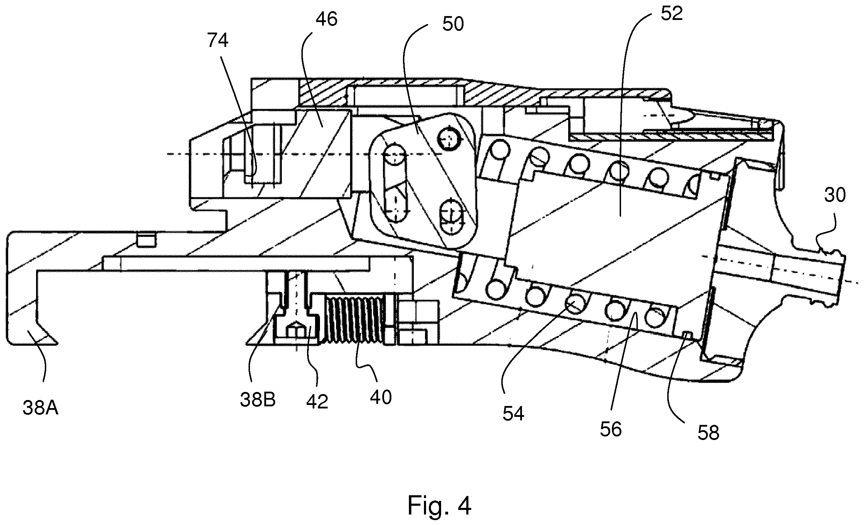

Der Befestigungsabschnitt 36 weist zwei zueinander weitenverstellbare hinterschnittene Klemmbacken 38a und 38b auf, mit welchen der Technikblock 12 reib- und formschlüssig an einer entsprechenden Schiene oder ähnlichen Haltevorrichtung befestigt werden kann. Die einstellbare Klemmbacke 38b ist wie aus der Fig. 4 ersichtlich über eine Feder 40 gegen die feste Klemmbacke 38a hin vorgespannt. Die verstellbare Klemmbacke 38b lässt sich ferner über eine Klemmschraube 42 fixieren.

Im Gehäuseabschnitt 32 befindet sich eine Zylinder-Kolben-Mechanik 44, welche einen wesentlichen Bestandteil des Spannmechanismus darstellt. Ein in dem Gehäuseabschnitt 32 in axialer Richtung, d.h. in Längsrichtung des Gliederarms 14, geführter Schieber oder Schlitten 46, der mit einem in dem Gliederarm 14 geführtes Zugseil 48 verbindbar ist, ist über ein Zwischengelenk 50 mit einem Ende eines Kolbens 52 der Zylinder-Kolben-Mechanik 44 gelenkig verbunden, so dass eine Hubbewegung des Kolbens 52 zu einer translatorischen, axialen Verschiebung des Schlittens 46 führt.

Der Kolben 52 wird über eine Spiraldruckfeder 54 in eine Richtung vorgespannt, in welche der Kolben 52 über den Schlitten 46 das Zugseil 48 zum Technikblock 12 hin zieht und auf diese Weise, wie weiter unter im Detail beschrieben wird, den Gliederarm verspannt und feststellt bzw. versteift. Aufgrund der Federvorspannung befindet sich der Gliederarm 14 im Ruhestand, d.h. ohne äußeren Eingriff, im festgestellten oder arretierten Zustand.

Um den an sich flexiblen Gliederarm 14 in beliebige Richtung biegen zu können, muss die Federvorspannung aufgehoben werden. Dies erfolgt mittels einer externen Energiequelle. Der Kolben 52 kann entgegen der Druckkraft der Spiraldruckfeder 54 mit Hilfe einer Druckluft, welche über den Druckluftanschluss 30 einem Zylinderraum 56 der Zylinder-Kolben-Mechanik 44 zugeführt wird, in die entgegengesetzte Richtung betätigt werden und dabei durch entsprechende Verschiebung des Schlittens 46 das Zugseil 48 freigeben oder entspannen und somit den festgestellten Gliederarm lösen.

Ein oder mehrere Kolbenringe 58 sorgen für die fluiddichte Trennung des mit Druckluft beaufschlagten Teils des Zylinderraums 56 von dem Teil des Zylinderraums 56, in dem sich die Spiraldruckfeder 54 befindet. Die Bewegung des Kolbens 52 bzw. des Schlittens 46 ist in beiden Richtungen jeweils durch Anschläge begrenzt, um definierte Verstellwege zum Lösen und Spannen vorzugeben.

Im Folgenden wird die Kopplung des Arbeitsmoduls B mit dem Technikmodul A detaillierter beschrieben.

Die Schnittstelle zwischen dem Arbeitsmodul B und dem Technikmodul B dient nicht nur zur rein mechanischen, sondern auch zur signaltechnischen und funktionalen Kopplung beider Einheiten, da zum einen die über das distale Betätigungselement 26 eingegebenen Steuersignale an den Technikblock 12 und von dort über die Steuersignalleitung 20b an die Adaptionseinheit 18 mit den entsprechenden Steuerorganen übertragen werden müssen und zum anderen das im Gliederarm 14 verlaufende Zugseil 48 mit der Zylinder-Kolben-Mechanik 44 gekoppelt werden muss.

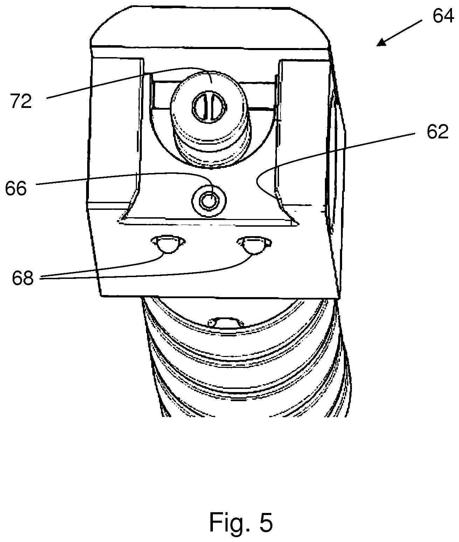

Zur mechanischen Kopplung des Moduls A mit dem Modul B weist der Kopplungsabschnitt 34 des Technikblocks 12, welcher sich unmittelbar oberhalb des Befestigungsabschnitts 36 befindet, eine sich vertikal erstreckende Schwalbenschwanzführung 60 auf, welche eine formschlüssige Verbindung mit einer komplementär ausgebildeten Führungsaufnahme 62 an einem Kopplungsabschnitt 64 des Arbeitsmodul B bildet (siehe Fig. 5). Hierfür wird der Kopplungsabschnitt 64 vertikal auf die Schwalbenschwanzführung 60 aufgeschoben, bis die beiden Kopplungsabschnitte 34 und 64 in flächige Anlage kommen. Zur lösbaren Arretierung beider Kopplungsabschnitte 34 und 64 ist am Kopplungsabschnitt 64 des Arbeitsmoduls B ein Verriegelungselement 66 in Form einer federvorgespannten Verriegelungsnase vorgesehen. Ferner sind an der Unterseite des Kopplungsabschnitts 64 zwei elektrische Kontakte 68 vorgesehen, die mit entsprechenden Kontaktstellen 70 am Kopplungsabschnitt 34 des Technikblocks 12 in Kontakt kommen, wenn sich die beiden Module A und B in ihrer gekoppelten und verriegelten Arbeitsposition befinden. Die elektrischen Kontakte 68 stehen über elektrische Leitungen 90 mit dem distalen Betätigungselement 26 in Verbindung. Im Technikblock 12 sind ebenfalls (nicht dargestellte) Leitungen vorgesehen, welche die Kontaktstellen 70 mit der ausgehenden Steuerleitung 20b verbinden.

Aus der Fig. 5 ist ferner ein Endstück 72 des Zugseils 48 zu erkennen. Bei dem Endstück 72 handelt es sich um ein rotationssymmetrisches profiliertes Drehteil, welches passgenau in eine entsprechende Ausnehmung 74 im Schlitten 46 passt und auf diese Weise in axialer Richtung bzw. in Zugrichtung formschlüssig mit dem Schlitten 46 verbindbar ist. Das Endstück 72 am freien Ende des Zugseils 48 stellt ferner sicher, dass das durch den Kopplungsabschnitt 64 geführte Zugseil 48 nicht ausfädelt.

Das Endstück 72 kann in die Ausnehmung 74 von oben eingelegt werden, wenn sich der Schieber 46 in der voll ausgefahrenen Position befindet. Da der Schlitten 46 im Ruhezustand aufgrund der Vorspannung der Feder 54 eingefahren ist, muss zum Einlegen des Endstücks 72 in die Ausnehmung 74 der Schlitten 46 über die Zylinder-Kolben-Mechanik 44 ausgefahren werden. Durch Betätigen des Betätigungselements 28 am Technikblock 12 wird dem Technikblock 12 Druckluft zugeführt und der Kolben 52 mit Druck beaufschlagt, so dass sich dieser entgegen der Federkraft der Feder 54 zum Gliederarm hin (in der Fig. 4 nach links) bewegt und dadurch den Schlitten 46 so weit aus dem Gehäuseabschnitt 32 schiebt, dass das freie Ende des Zugseils 48 bzw. Endstück 72 in die Ausnehmung 74 des Schlittens 46 eingelegt werden kann. Nach dem Loslassen des Betätigungselements 28 wird die Druckluftzufuhr unterbunden. Die Spiraldruckfeder 54 bewegt den Kolben 52 wieder in seine Ruheposition, wodurch der Schlitten 46 zusammen mit dem Endstück 72 des Zugseils 48 eingefahren wird. Durch das Einfahren des Schlittens 46 wird ferner das Zugseil 48 gespannt und der Gliederarm 14 in seiner momentanen Position fixiert und festgestellt. In der eingefahrenen Stellung befindet sich der Schlitten 46 soweit innerhalb des Gehäuseabschnitts 32, dass das Endstück 72 in der Ausnehmung 74 des Schlittens 46 gesichert ist.

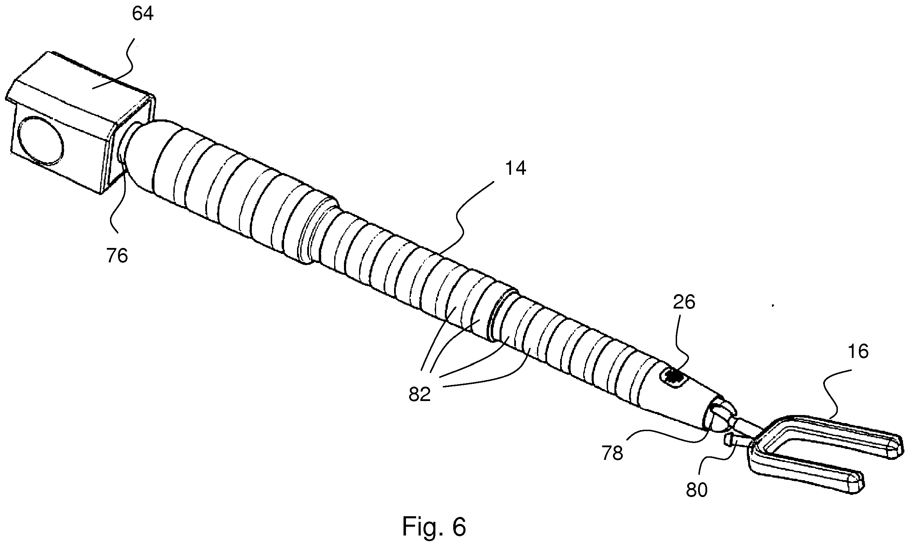

In der Fig. 6 ist das gesamte Arbeitsmodul B, bestehend aus dem Kopplungsabschnitt 64, einem proximalen Gelenk 76, dem eigentlichen Gliederarm 14, dem distalen Betätigungselement 26, welches am distalen Ende des Gliederarms 14 angeordnet ist, einem distalen Schwenkgelenk 78 und dem Halteelement 16. Das proximale (Kugel-)Gelenk 76 erlaubt eine Drehung des Gliederarms 14 um die Längsachse bezüglich des Kopplungsabschnitts 64 und ferner eine Schwenkbewegung des Gliederarms 14, um den Gliederarm 14 vertikal in die Körperhöhle des Patienten absenken zu können. Das distale Schwenkgelenk 78 ermöglicht eine Schwenkbewegung des Halteelements 16 bezüglich des distalen Endes des Gliederarms 14. Das Halteelement 16 kann beispielsweise als vakuumunterstütztes Stabilisierungs- oder als Positionierungselement ausgeführt sein. Handelt es sich um ein Stabilisierungselement, so wird der vakuumunterstützte U-förmige Teil mitsamt dem Gliederarm 14 arretiert, sobald Zugkraft auf das Zugseil 48 gebracht wird. Die Arbeitsenden des Halteelements 16 werden meist aus weichen Materialen ausgeführt, um sich ans Gewebe oder auch anatomischen Gegebenheiten ideal anzupassen und dadurch so wenig Leckagen des Vakuums wie möglich zuzulassen. Das Halteelement 16 kann sich mittels Unterdruck am Gewebe bzw. Organ ansaugen. Zur Bereitstellung des Unterdrucks weist das Halteelement 16 einen Anschluss 80 auf, so dass das Halteelement 16 über eine nicht dargestellte Unterdruckleitung mit Unterdruck beaufschlagt werden kann. Das Halteelement 16 kann in einem anderen Ausführungsbeispiel mit einem universellen Adapter versehen sein, um den Anschluss verschiedenster einmal- oder wiederverwendbarer chirurgischer Instrumente und Geräte, die mittels des Gliederarms 14 während eines chirurgischen Eingriffs gehalten und positioniert werden, zu ermöglichen.

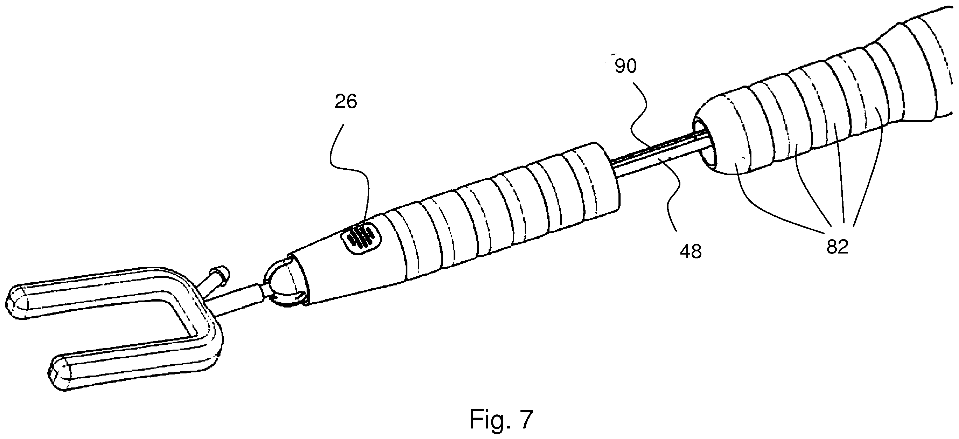

Durch den Gliederarm 14 verläuft ein Zugseil 48, wie dies in der Fig. 7 gezeigt ist, bei der aus Darstellungsgründen einige der Gliederelemente 82 des Gliederarms 14 fehlen. Der flexible Gliederarm 14 besteht aus einer Vielzahl an rotationssymmetrischen einzelnen Gliederelementen 82, welche mit Ausnahme der Größenordnung baugleich sind und wie eine Vielzahl aneinander gereihter Kugelgelenke wirken. Die Gliederelemente 82 haben im Wesentlichen die Form einer Kugelhülse oder einer Kugelkalotte mit einer zentralen Durchgangsöffnung 84, einem distalen sphärischen Außenoberfläche 86 und einen proximalen sphärischen Innenoberfläche 88. Benachbarte Gliederelemente 82 stehen an diesen sphärischen Flächenabschnitten 86, 88 in Kontakt bzw. werden über das Zugseil 48 in Kontakt gehalten.

Die Gliederelemente können beispielsweise im Spritzgussverfahren kostengünstig hergestellt werden. Dabei sind in Bezug auf Stauchung sehr steife Materialien vorzusehen. Um den Reibkoeffizienten zwischen den aneinander abgleitenden Gliederelementen 82 zu erhöhen, können auch Materialkombinationen verwendet werden. Insbesondere können die aneinander abgleitenden sphärischen Flächen 86, 88 entsprechend beschichtet sein.

Über das mittig durchgeführte Zugseil 48, welches mit dem letzten Gliederelement 82 bzw. distalen Schwenkgelenk 78 auf der distalen Seite gekoppelt ist, lassen sich die Gliederelemente 82 gegen das das erste Gliederelement 82 bzw. das proxximale Gelenk 76 auf der proximalen Seite verspannen. Durch den Kraft- bzw. Reibschluss zwischen den sphärischen Flächen 86, 88 können die einzelnen Gliederelemente 82 in nahezu jeder beliebigen Relativposition zueinander fixiert werden, wodurch der Gliederarm 14 festgestellt oder versteift wird. Gleichzeitig werden dadurch das proximale Gelenk 76 und das distale Gelenk 78 arretiert. Wird dagegen das Zugseil 48 entspannt, löst sich der Reibschluss zwischen den einzelnen Gliederelementen 82, so dass diese wieder aneinander abgleiten und sich jeweils relativ zueinander bewegen können.

Aus der Fig. 6 ist ferner erkennbar, dass die Gliederelemente 82 am proximalen Ende des Gliederarms 14 größer als die Gliederelemente 82 am distalen Ende des Gliederarms 14 sind. Der Grund hierfür ist wie folgt. J je weiter distal sich ein Gliederelement 82 befindet, desto geringer ist das Biegemoment, welches bei Auftreten von Querkraft auf dieses Gliederelement 82 wirkt. Da das Biegemoment, wenn Querkraft am Arm eingeleitet wird, an den proximalen Gliederelemente 82, d.h. an den Gliederelementen mit dem größten Abstand zum distalen Ende, am größten ist, werden diese sich zuerst gegeneinander verschieben. Durch entsprechende Wahl unterschiedlicher Durchmesser der Gliederelemente 82 entsprechend dem zu erwartenden Biegemomentenverlauf kann eine einheitliche Biegung des Arms erreicht werden.

Das distale Betätigungselement 26, welches in dieser Ausführungsform als elektrischer Taster ausgebildet ist, ist über zwei elektrische Leitungen 90, welche ebenfalls innen durch die Gliederelemente 82 geführt sind und welche die eingegebenen Steuersignale an die elektrische Kontakte 68 im Kopplungsabschnitt 64 übertragen. Da die Leitungen 90 innerhalb der Gliederelemente 82 verlaufen, sind sie vor äußeren Einflüssen geschützt. Jedoch muss dafür gesorgt werden, dass die Leitungen 90 nicht durch die Bewegung der einzelnen Gliederelemente 82 bzw. des Zugseils 48 verletzt oder beschädigt werden.

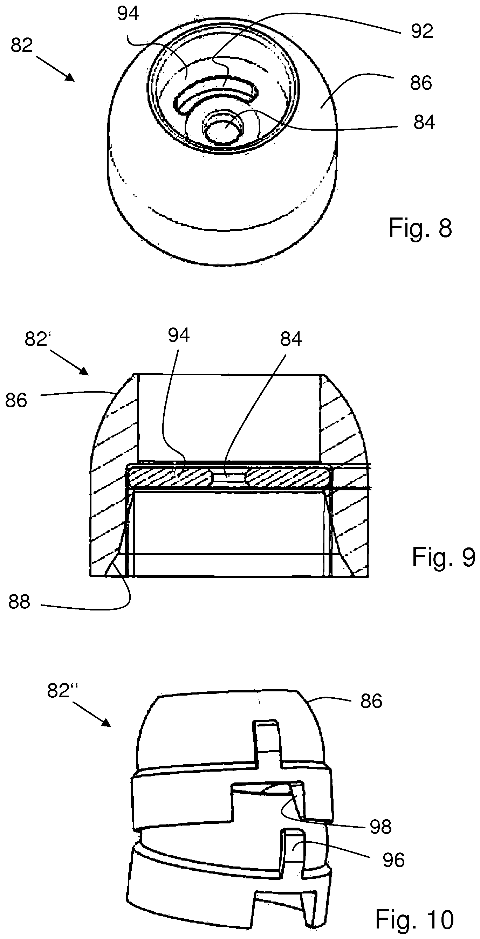

Vor diesem Hintergrund weisen die einzelnen Gliederelemente 82, wie in der Fig. 8 gezeigt ist, neben der zentralen Durchgangsöffnung 84 für das Zugseil 48 eine oder mehrere, außermittig angeordnete Aussparungen 92 auf, durch welche Energieübertragungsleitungen 90, wie Schläuche, Kabel, oder ähnliches geschützt vor der Bewegung des parallelen Zugseils 48 verlaufen.

Da sich die einzelnen Gliederelemente 82 nicht nur seitlich zueinander verschieben oder verkippen lassen, sondern auch zueinander verdrehen können, könnte dies zu einer Verdrehung und gegebenenfalls Beschädigung der Leitungen 90 führen. Vor diesem Hintergrund befinden sich bei den in der Fig. 9 gezeigten Gliederelementen 82' gemäß einer zweiten Ausführungsform die zentrale Durchgangsöffnung 84 und die Aussparungen 92 für die Leitungen 90 in einer Drehscheibe 94, welche um die Längsachse drehbar in den Gliederelementen 82' aufgenommen ist. Diese Drehscheiben 94 können somit eine Verdrehung der Gliederelemente 82 ausgleichen.

Eine dritte Ausführungsform der Gliederelemente 82" ist in der Fig. 10 dargestellt. Fig. 10 zeigt zwei Gliederelemente 82", die zur Verdrehsicherung der Gliederelemente 82'" mit formschlüssigen Eingriffselementen 96, 98 versehen sind. Bei den Eingriffselementen 96, 98 handelt es sich zum einen um Vorsprünge 96, die im Bereich der sphärischen Außenoberfläche 86" ausgebildet sind, und zum anderen um Ausbrüche 98 im Bereich der sphärischen Innenoberfläche 88", wobei die Vorsprünge 96 in die Ausbrüche 98 eingreifen. Wie in der Fig. 10 zu sehen ist, ist die Breite der Ausbrüche 98 größer als die der Vorsprünge 96, so dass ein gewisser rotatorischer Freiheitsgrad zugelassen wird und sich der Vorsprung 96 innerhalb des Ausbruchs 98 in Drehrichtung etwas bewegen kann.

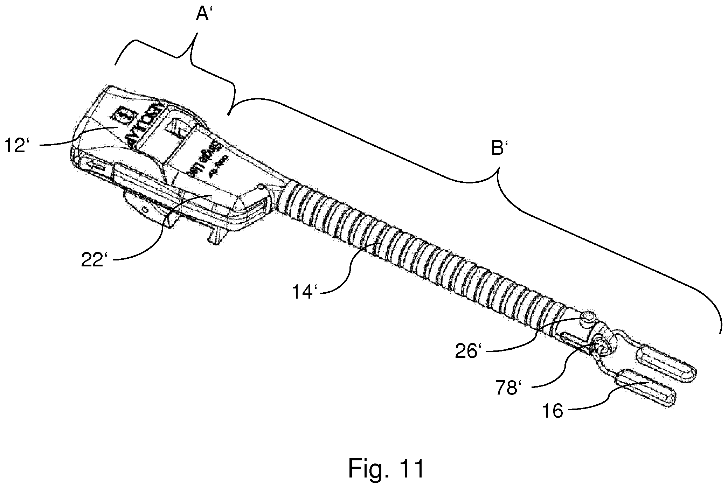

Fig. 11 zeigt eine perspektivische Ansicht der chirurgischen Vorrichtung 10' gemäß einer nicht separat beanspruchten vierten Ausführungsform. Der wesentlichste Unterschied zu den oben beschriebenen Ausführungsformen ist die integrale Einbindung der Energiequelle in Form einer integrierten Druckluftkartusche 22' in dem vergrößerten Kopplungsabschnitt 64' des Gliederarms 14'.

Funktional gewährleistet der Kopplungsabschnitt 64' zum einen die gleiche Koppelbarkeit des Gliederarms 14' und des Technikblocks 12' bezüglich des Zugseils 48' und der Zylinder-Kolben-Mechanik 44' bzw. bezüglich des Endstücks 72' und der Ausnehmung 74' sowie bezüglich der elektrischen Kontakte 68' und der Kontaktstellen 70', wie der Gliederarm14 und der Technikblock 12 gemäß den ersten Ausführungsformen der Erfindung. Zum anderen weist aber eine Chirurgische Vorrichtung 10' gemäß der vierten Ausführungsform darüber hinaus auch Komponenten am Gliederarm 14' und am Technikblock 12' auf, die die Anbindung der integrierten Druckkartusche 22' an der Zylinder-Kolben-Mechanik 44' sicherstellen.

Die konkrete Ausgestaltung der vierten Ausführungsform unterscheidet sich im distalen Bereich des Arbeitsmoduls B' in der Umsetzung des Schwenkgelenks 78' zwischen dem gabelförmigen Halteelement 16 und dem Gliederarm14' dahingehend, dass die Schwenkachse orthogonal verdreht im Vergleich zu den ersten Ausführungsformen verläuft. Es wird also eine Nickbewegung des Halteelements 16 anstelle einer Gierbewegung zugelassen.

Das distale Betätigungselement 26' ist zur leichteren Handhabung bezüglich der unmittelbaren Umgebung des Betätigungselements 26' hervorgehoben bzw. exponiert.

Im Gegensatz zu den ersten drei Ausführungsformen ändern sich die Außenabmaße der Gliederelemente 82'" des Gliederarms 14' der vierten Ausführungsform über die Länge des Gliederarms 14' nicht. Um trotzdem eine dem Biegemomentenverlauf entsprechende gleichmäßige Biegsamkeit des Gliederarms 14' zu erreichen, werden jeweilige Kegelwinkel α (siehe Fig. 9) der Gliederelemente 82'" vom Technikblock 12' aus in Richtung des Halteelements 16 graduell (d.h. individuell für jedes Gliederelement 82'" angepasst) oder abschnittsweise (d.h. für benachbarte Gliederelemente 82'" in einem Abschnitt des Gliederarms 14' gleich) vergrößert.

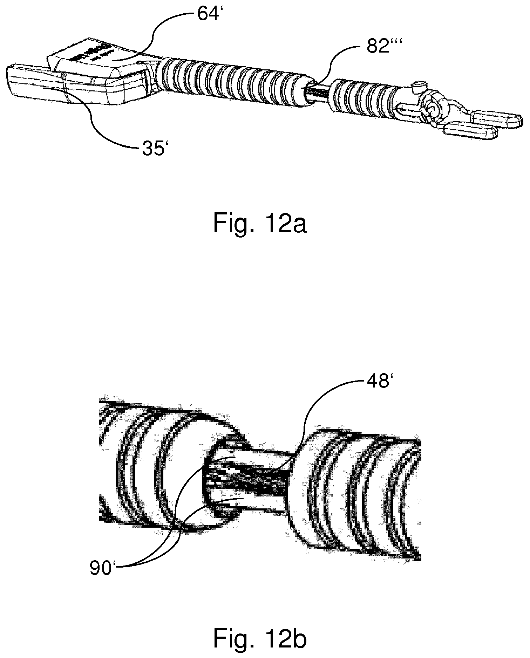

Wie in den Figuren 12a und 12b gezeigt, in denen zur Veranschaulichung einzelne Gliederelemente 82'" nicht dargestellt sind, verläuft im Zentrum des Gliederarms 14' das Zugseil 48' und verlaufen drei über den Umfang des Zugseils 48' gleichmäßig verteilte elektrische Leitungen 90' im Inneren des Gliederarms 14'.

In dem Bereich des Kopplungsabschnitts 64' des Arbeitsmoduls B', der mit Gliederarm 14' fluchtet, werden sowohl das Zugseil 48' als auch die elektrischen Leitungen 90' von der Vorderseite des Kopplungsabschnitts 64' vom Gliederarm 14' aus in Richtung der Rückseite des Kopplungsabschnitts 64' zu den entsprechenden Anschlussstellen der elektrischen Kontakte 68' und des Endstücks 72' geleitet.

Das Endstück 72' ist T-förmig ausgestaltet, wobei der Querbalken des T-förmigen Endstücks 72' vorzugsweise quer zu der Richtung ist, in der bei der Montage der Kopplungsabschnitt 64' des Arbeitsmoduls B' in den Kopplungsabschnitt 34' des Technikmoduls A' eingelegt wird.

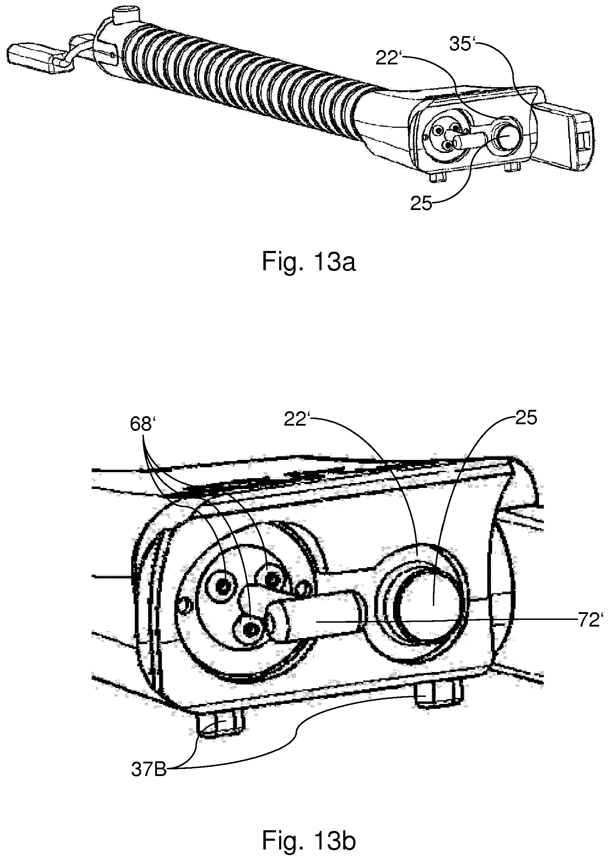

In dem Bereich des Kopplungsabschnitts 64' des Arbeitsmoduls B', der nicht mit dem Gliederarm 14' fluchtet, ist die integrierte Druckluftkartusche 22' untergebracht. Wie aus den Figuren 13a und 13b hervorgeht, ist die Druckluftkartusche 22' derart im Kopplungsabschnitt 64' des Arbeitsmoduls B' angeordnet, dass an der Rückseite des Kopplungsabschnitts 64' eine Anschlussstelle der Druckluftkartusche 22' in Form einer versiegelnden Membran 25 zugänglich ist. Die Druckkartusche 22' kann durch Schwenken eines Anschlusshebels 35 axial, d. h. im Wesentlichen senkrecht bzw. quer zur Rückseitenoberfläche des Kopplungsabschnitts 64', bewegt werden.

Die Rückseitenoberfläche des Kopplungsabschnitts 64' ist derart kreiszylindrisch geformt bzw. gekrümmt, dass sie im unteren Bereich im Wesentlichen senkrecht zum gerade ausgerichteten Gliederarm 14' und im oberen Bereich angewinkelt dazu verläuft.

An der Unterseite des Kopplungsabschnitts 64' in der Nähe der Rückseitenoberfläche des Kopplungsabschnitts 64' ragen zwei Kopplungsvorsprünge 37B nach unten vor.

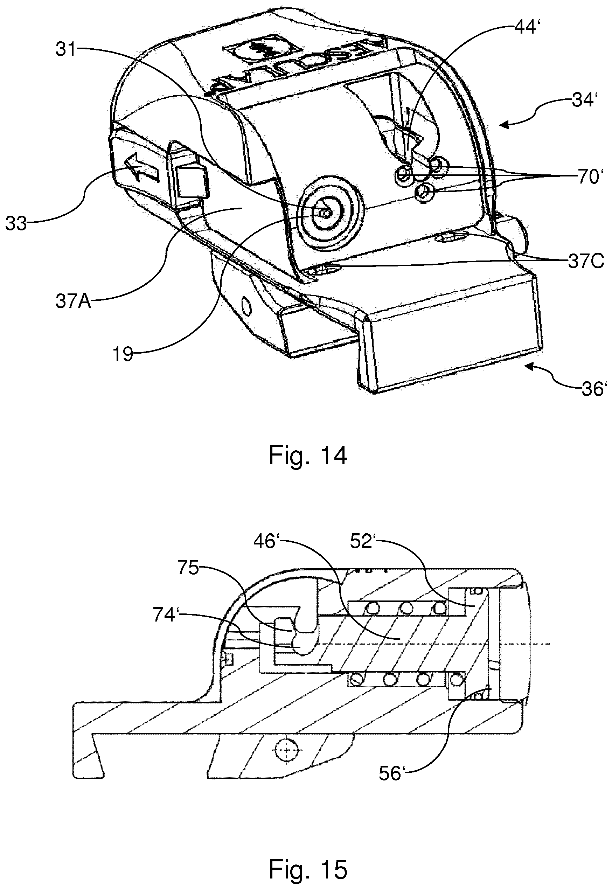

Fig. 14 zeigt eine perspektivische Ansicht der Vorderseite des Technikmoduls A' der chirurgischen Vorrichtung 10' gemäß der vierten Ausführungsform, an die die Rückseite des Arbeitsmoduls B' ankoppelbar ist.

Entsprechend dem mit dem Gliederarm 14' fluchtenden Bereich des Koppelungsabschnitt 64' des Arbeitsmoduls B' sind an dem mit dem Gliederarm 14' fluchtenden Bereich des Koppelungsabschnitt 34' des Technikmoduls A' Anschlussstellen in Form der Kontaktstellen 70' und in Form der Ausnehmung 74' vorgesehen, die bei der Montage des Arbeitsmoduls B' an dem Technikmodul A' mit den elektrischen Kontakten 68' und dem Endstück 72' verbunden werden.

An der Vorderseite des mit dem Gliederarm 14' nicht fluchtenden Bereichs des Kopplungsabschnitts 34' des Technikmoduls A' ist ein Druckluftanschluss mit einem Dorn 31 und einer Druckregelungseinheit 19 angeordnet.

Der Befestigungsabschnitt 36' des Technikmoduls A' entspricht im Wesentlichen dem Befestigungsabschnitt 36 der ersten drei Ausführungsformen ragt aber nach vorne so aus, dass der Koppelungsabschnitt 64' des Arbeitsmoduls B' nicht nur an der Vorderseite des Technikmoduls A' anliegt, sondern auch auf Teilen der Oberseite des Befestigungsabschnitts 36' des Technikmoduls A' aufliegt. An der Oberseite des Befestigungsabschnitts 36' sind zwei Koppelungsaussparungen 37C vorgesehen.

Seitlich am Technikblock A' ist eine Hebelaussparung 37A so ausgeformt, dass sich der am Arbeitsmodul B' schwenkbar angebrachte Anschlusshebel 35 nur dann in die Hebelaussparung 37A einklappen lässt, wenn sich das Technikmodul A' und das Arbeitsmodul B' in einer definierten Stellung relativ zueinander zum Ankoppeln befinden. Um den Anschlusshebel 35 in seiner eingeklappten Stellung zu verrasten, ist ein Verrastungsschieber 33 zum Arretieren des Anschlusshebels 35 vorgesehen.

Die Vorderseitenoberfläche des Kopplungsabschnitts 34' des Technikmoduls A' ist mit der Rückseitenoberfläche des Koppelungsabschnitts 64' des Arbeitsmoduls B' abgestimmt, weist also im Wesentliche die gleiche kreiszylindrische Krümmung wie die Rückseitenoberfläche des Koppelungsabschnitts 64' auf.

Fig. 15 zeigt eine Querschnittsansicht des Technikblocks 12' der chirurgischen Vorrichtung 10' gemäß der vierten Ausführungsform. Wie auch ein Vergleich mit Fig. 4 verdeutlicht, sind bei der vierten Ausführungsform der Schlitten 46' und der Kolben 52' koaxial und einstückig ausgebildet. Ein verbindendes Zwischengelenk 50 ist gemäß dieser Ausführung nicht erforderlich.

Die Ausnehmung 74' im Schlitten 46' weist eine Klemmnase 75 auf, die so geformt ist, dass die Zylinder-Kolben-Mechanik 44' beim Einführen des Endstücks 72' in die Ausnehmung 74' durch das Einführen in axialer Richtung des Kolbens 52' verschoben werden kann. Beim Einführen des Endstücks 72' in die Ausnehmung 74' wird die Zylinder-Kolben-Mechanik 44' bei gleichzeitiger Überwindung der von der Spiraldruckfeder 54 ausgeübten Kraft bis zu einem bestimmten Wendepunkt in Richtung hin zum Gliederarm 14' verschoben, bevor die Zylinder-Kolben-Mechanik 44' nach Überwindung des Wendepunkts unter Zuhilfenahme der von der Spiraldruckfeder 54 ausgeübten Kraft in Richtung weg vom Gliederarm 14' verschoben wird. Mittels der Klemmnase 75 kann also die Spannung des Gliederarms 14' bereits beim Montieren des Arbeitsmoduls B' erzeugt werden. Die Klemmnase 75 kann so ausgeformt sein, dass das Endstück 25 nur nach Verstellen der Zylinder-Kolben-Mechanik 44' durch Betätigung des distalen Betätigungselements 26' wieder aus der Ausnehmung entnommen werden kann, dass ein Entnehmen des Endstücks 72' also durch Selbsthemmung verhindert wird. Andernfalls kann die Ausnehmung 74', wie in Fig. 15 gezeigt, auch so ausgeformt sein, dass durch bloßes Anheben des Kopplungsabschnitts 64' des Arbeitsmoduls B' die Spannung der Spiraldruckfeder 54 überwunden und so das Endstück 72' der Ausnehmung 74' entnommen werden kann. In diesem Fall dient die Ansteuerbarkeit des Spannmechanismus (44', 48') mithilfe der Energiequelle in Form der Druckluftkartusche 22' nur dazu, den Gliederarm 14' in einem montierten Zustand zu verstellen.

Die Montage der erfindungsgemäßen Vorrichtung 10 gemäß den ersten drei Ausführungsformen ist wie folgt:
Zunächst wird der Technikblock 12 über Druckluftleitungen 20a und Druckluftleitungen mit der Adaptionseinheit 18 verbunden. An der Adaptionseinheit wird die Druckluftquelle, z.B. einen Druckluftkartusche 22, angeschlossen. Anschließend wird der Technikblock 12 über den Befestigungsabschnitt 36 an einer Halterung in der Nähe des Operationsfeldes, z.B. an einen Sternumspreizer, befestigt. Anschließend oder bereits zuvor wird das Arbeitsmodul B mit dem Technikmodul A bzw. Technikblock 12 verbunden. Hierfür wird das proximale Betätigungselement 28 gedrückt, so dass der Schlitten 46 aus dem Gehäuseabschnitt 32 herausfährt. Wenn nun der Kopplungsabschnitt 64 des Arbeitsmoduls B mit dem Kopplungsabschnitt 34 des Technikmoduls A verbunden wird, stellen die Schwalbenschwanzführung 60 und die Aufnahme 62 einen Formschluss zwischen beiden Modulen A und B her. Ferner wird die elektrischen Verbindung zwischen den Kontakten 68 und 70 herstellt. Darüber hinaus wird das Zugseil 48 mit der Zylinder-Kolben-Mechanik 44 gekoppelt, da beim Aufschieben der Kopplungsabschnitts 64 gleichzeitig das vorspringende Endstück 72 des Zugseils 48 in die entsprechende Ausnehmung 74 des Schlittens 46 eingelegt wird. Wird nun das proximale Betätigungselement 28 wieder losgelassen, zieht sich der Schlitten 46 wieder in das Gehäuse 32 zurück und spannt hierbei das Zugseil 48 und fixiert den Gliederarm 14 und das Haltelement 16 in ihren jeweiligen Positionen und Lagen.

Die Montage der erfindungsgemäßen Vorrichtung 10 gemäß der vierten Ausführungsform ist wie folgt:
Zunächst wird der Technikblock 12' über den Befestigungsabschnitt 36' an einer Halterung in der Nähe des Operationsfeldes, z.B. an einen Sternumspreizer, befestigt. Anschließend oder bereits zuvor wird das Arbeitsmodul B' mit dem Technikmodul A' bzw. Technikblock 12' verbunden. Hierfür wird das proximale Ende des Gliederarms 14' bzw der Kopplungsabschnitt 64' mit dem Anschlusshebel 35 in seiner geöffneten Position so an den Kopplungsabschnitt 34' des Technikblocks 12' herangeführt, dass die Kopplungsvorsprünge 37B in die Kopplungsaussparungen 37C einfahren.

Wie Fig. 15 zu entnehmen ist, fährt dabei gleichzeitig das Endstück 72' des Zugseils 48' in die Ausnehmung 74' der Zylinder-Kolben-Mechanik 44' ein. Die Klemmnase 75 bewirkt, dass das Endstück 72' beim Einfahren in die Ausnehmung 74' in proximale Richtung gezogen und damit der Gliederarm 14' gespannt wird.

Erst wenn sich der Kopplungsabschnitt 64' des Arbeitsmoduls B' mit dem geöffneten Anschlusshebel 35 nach Einfahren der Kopplungsvorsprünge 37B am Anschlag befindet, kann der Anschlusshebel 35 in die dafür vorgesehene Hebelaussparung 37A und damit in seine geschlossene Stellung geschwenkt werden.

Die integrierte Druckluftkartusche 22' ist derart im proximalen Ende des Gliederarms 14' angeordnet, dass deren Anschluss bei der Montage des Gliederarms 14' in Richtung des Technikblocks 12' zeigt. Der Druckkluftanschluss der integrierten Druckluftkartusche 22' wird durch die versiegelnde Membran 25 gebildet, die für die Zufuhr von Druckluft von der integrierten Druckluftkartusche 22' an den Technikblock 12' durchstoßen werden muss. Sobald die Membran 25 einmal durchstoßen ist, kann die integrierte Druckluftkartusche 22' nicht mehr oder zumindest nicht ohne erheblichen Aufwand wieder luftdicht verschlossen geschweige denn wieder mit Druckluft befüllt werden.

Bei der vierten Ausführungsform erfolgt die Perforation der Membran 25 mithilfe des am Kopplungsabschnitt 34' des Technikblocks 12' angeordneten bewegbaren Dorns 31. Sind der Kopplungsabschnitt 64' des Gliederarms 14' und der Kopplungsabschnitt 34' des Technikblocks 12' in einer dafür vorgesehenen relativen Stellung zueinander, kann mittels des Anschlusshebels 35 der Abstand zwischen Dorn 31 und Membran 25 bis zur Kollision und damit zur Perforation verringert werden.

Der Anschlusshebel 35 kann sich wie abgebildet am Gliederarm 14' befinden und kann lediglich die integrierte Druckluftkartusche 22' und damit die Membran 25 in Richtung des Dorns 31 bewegt werden. Alternativ wäre es aber auch möglich, den Anschlusshebel 35 am Technikblock 12' vorzusehen, nur den Dorn 31 in Richtung der Membran 25 bewegbar zu gestalten oder sowohl den Dorn 31 als auch die Membran 25 bewegbar zu gestalten.

Mit dem Anschlusshebel 35 wird nicht nur dafür gesorgt, dass die Membran 25 durchstoßen wird, sondern wird auch die Anbindung der integrierten Druckluftkartusche 22' an die Zylinder-Kolben-Mechanik 44' über die Druckregelungseinheit 19 umgesetzt.

Unmittelbar bevor die Membran 25 perforiert wird, wird der Bereich um die Perforationsstelle nach außen hin abgedichtet. Dies wird dadurch bewerkstelligt, dass die zum Abdichten vorgesehenen Komponenten und die für die Perforation der Membran 25 beweglich ausgestalteten Komponenten (Membran 25 und/oder Dorn 31) unterschiedlich so gelagert werden, dass die zum Abdichten vorgesehenen Komponenten und die für die Perforation der Membran 25 beweglich ausgestalteten Komponenten zwar beide vom Anschlusshebel 35 angesteuert werden können, dass aber die zum Abdichten vorgesehenen Komponenten den für die Perforation der Membran 25 beweglich ausgestalteten Komponenten vorauseilen.

Die Arbeitsweise der erfindungsgemäßen Vorrichtung 10 ist wie folgt:
Durch Betätigen des distalen Betätigungselements 26 werden über entsprechende elektrische Signale die Druckluftventile in der Adaptionseinheit 18 oder im Technikblock 12 angesteuert, welche, bei den ersten drei Ausführungsformen über die Druckluftleitungen 20a, den Kolben 52 in dem Technikblock 12 mit Druck beaufschlagen und das Zugseil 48 entspannen. Somit kann der Operateur mit einer Hand den Gliederarm 14 am distalen Ende greifen, entsprechend verformen und das Haltelement 16 in die Körperhöhle an das zu stabilisierende Gewebe oder zu haltende Organ führen. Lässt der Operateur nun das distale Betätigungselement los oder betätigt es ein weiteres Mal, wird die externe Energiezufuhr zum Spannmechanismus bzw. die Druckluftzufuhr zur Zylinder-Kolben-Mechanik 44 unterbunden und über die Spiraldruckfeder 54 der Spannmechanismus wieder gespannt und dadurch Gliederarm 14 und Haltelement 16 in ihrer momentanen Stellung fixiert oder festgestellt.

Durch nochmaliges Betätigen des distalen Betätigungselements 26 oder proximalen Betätigungselements 28 kann der Spannmechanismus zum Nachjustieren oder Entfernen des Gliederarms 14 aus der Körperhöhle wieder gelöst werden.

Wird bei einer chirurgischen Vorrichtung 10' gemäß der vierten Ausführung der Gliederarm 14' nach der erstmaligen Montage wieder demontiert, bewirkt das Öffnen des Anschlusshebels 35, dass zunächst die Abdichtung der Membran-Druckregelungseinheit-Verbindung aufgegeben wird. Dadurch entweicht die restliche zuvor nicht verbrauchte Druckluft aus der integrierten Druckluftkartusche 22'. Folglich kann der Gliederarm 14' nach einer zweiten Montage nicht mehr mithilfe der Zylinder-Kolben-Mechanik 44 verstellt werden und verliert somit einen Großteil seiner Funktionalität.

Die vorliegende Erfindung ist jedoch nicht auf die zuvor detailliert beschriebenen Ausführungsformen beschränkt, sondern kann innerhalb des Schutzbereichs der beigefügten Ansprüche variiert werden. Im Folgenden sind einige solcher Variationsmöglichkeiten aufgeführt.

Anstelle des modularen Aufbaus von Arbeitsmodul B und Technikmodul B können diese einteilig aufgebaut und insgesamt als einmalverwendbare Komponente vorgesehen sein. Damit entfallen alle Schnittstellen zwischen beiden Einheiten und die damit verbundenen Arbeitsschritte zum Konnektieren. Dieses Konzept kann wiederum entweder mit nur einem, idealerweise distal angeordneten, oder zwei Betätigungselementen zur Steuerung des externen Energieflusses ausgeführt werden.

Gemäß der ersten Ausführungsform stammt die Druckluft von einer Druckluftkartusche 22, welche ein völlig autonomes Arbeiten ermöglicht. Anstelle der Druckluftkartusche 22 kann an dem Druckluftanschluss 24 der Adaptionseinheit 18 ein Druckluftschlauch angeschlossen und mit einer beliebigen anderen Druckluftquelle, z.B. einer vorhandenen Luftversorgung im Operationssaal, verbunden werden.

Die Energieübertragungsleitungen 90 zwischen dem distalen Betätigungselement 26 und dem Technikblock 12 können auch außerhalb des Gliederarms 14 verlaufen. Die Unterdruckleitung zum Haltelement 16 kann ebenfalls innerhalb des Gliederarms 14 verlaufen, wobei dann entsprechende Schnittstellen zwischen den Modulen A und B und Leitungen und Anschlüsse im Technikblock 12 vorzusehen sind.

Die Fluidsteuerungselemente in der Adaptionseinheit 18 können statt elektrisch auch pneumatisch angesteuert werden, wobei der Druckluftkolben 52 mit dem Arbeitsmedium Luft, z.B. bei einem Druck von 8 bar, betrieben wird, während das Ventil, welches den Energiefluss freigibt, mit einem wesentlich geringeren Druck angesteuert werden kann. Dieser geringe Druck sorgt wiederum für dünnwandigere, flexiblere Steuerleitungen und ein wesentlich kompakteres Betätigungselement.

Die Fluidsteuerungselemente können auch im Technikblock 12 vorgesehen sein und die Druckluftquelle direkt mit dem Technikblock 12 verbunden werden.

Beschrieben wurde eine chirurgische Vorrichtung zum Stabilisieren oder Immobilisieren von bewegtem Gewebe oder zum Positionieren von Organen, insbesondere eines Teils eines schlagenden Herzens, mit einem an einem Grundkörper befestigten oder befestigbaren flexiblen Arms4'), insbesondere Gliederarms, der in unterschiedliche Positionen und/oder Lagen bringbar ist und an dessen freien Ende zumindest ein Halteelement (16) angeordnet ist, und einem Spannmechanismus (44, 48; 44', 48'), über welchen der Arm in einer gewünschten Stellung feststellbar ist. Das Spannen und/oder Lösen des Spannmechanismus (44, 48; 44, 48') erfolgt mittels einer manuell ansteuerbaren externen Energiequelle (22; 22').

### Bezugszeichenliste

- 10, 10': Vorrichtung
- 12, 12': Grundkörper/Technikblock
- 14, 14': Gliederarm
- 16: Halteelement
- 18: Adaptionseinheit
- 19: Druckregelungseinheit
- 20: Leitungen
- 20a: Druckluftleitung
- 20b: Steuerleitung
- 22: Druckluftkartusche
- 22': integrierte Druckluftkartusche
- 24: Druckluftanschluss
- 25: Membran
- 26, 26': erstes Betätigungselement
- 28: zweites Betätigungselement
- 30: Druckluftanschluss
- 31: Dorn
- 32, 32': Gehäuseabschnitt
- 33: Verrastungsschieber
- 34, 34': Kopplungsabschnitt
- 35: Anschlusshebel
- 36, 36': Befestigungsabschnitt
- 37A: Hebelaussparung
- 37B: Kopplungsvorsprung
- 37C: Kopplungsaussparung
- 38A, 38B: Klemmbacken
- 40: Feder
- 42: Klemmschraube
- 44, 44': Zylinder-Kolben-Mechanik
- 46: Schlitten
- 48, 48': Zugseil
- 50: Zwischengelenk
- 52, 52': Kolben
- 54: Spiraldruckfeder
- 56, 56': Zylinderraum
- 58: Kolbenring
- 60: Schwalbenschwanzführung
- 62: Führungsaufnahme
- 64, 64': Kopplungsabschnitt
- 66: Verriegelungsnase
- 68, 68': elektrische Kontakte
- 70, 70': Kontaktstellen
- 72, 72': Endstück
- 74, 74': Ausnehmung
- 75: Klemmnase
- 76: Drehgelenk
- 78, 78': Schwenkgelenk
- 80: Anschluss
- 82; 82'; 82"; 82'": Gliederelemente
- 84: zentrale Durchgangsöffnung
- 86: sphärische Außenoberflächen
- 88: sphärische Innenoberflächen
- 90, 90': elektrische Leitungen
- 92: Aussparungen
- 94: Drehscheibe
- 96: Eingriffselemente
- 98: Ausbrüche

- A; A': Technikmodul
- B; B': Arbeitsmodul

- α: Kegelwinkel

## Patentansprüche

1. Chirurgische Vorrichtung (10) zum Stabilisieren oder Immobilisieren von bewegtem Gewebe, beispielsweise eines Teils eines schlagenden Herzens, oder zum Positionieren von Organen oder zum Positionieren und Halten von chirurgischen Instrumenten und Geräten während eines chirurgischen Eingriffs, mit:
einem Grundkörper (12), der über einen Befestigungsabschnitt (36) an einer im oder am Operationsfeld befindlichen Haltevorrichtung, insbesondere einem Sternumspreizer, befestigbar ist;
einem an dem Grundkörper (12) befestigbaren flexiblen Arm (14), insbesondere Gliederarm, der in unterschiedliche Positionen und/oder Lagen bringbar ist und an dessen freien Ende zumindest ein Halteelement (16) angeordnet ist;
einer manuell ansteuerbaren Energiequelle (22); und
einem Spannmechanismus (44, 48), über welchen der Arm in einer gewünschten Stellung feststellbar ist, und welcher aufweist:
ein Zugseil (48), das durch eine Vielzahl von zueinander beweglichen, insbesondere komplementär ausgebildeten, Gliederelementen (82; 82'; 82") des Arms (14) geführt ist und über welches die Gliederelemente (82; 82'; 82") reibschlüssig gegeneinander verspannt werden können; und
eine über die Energiequelle (22) betätigbare Zylinder-Kolben-Mechanik (44) zum Betätigen des Zugseils (48),
**dadurch gekennzeichnet, dass**
die Vorrichtung (10) modular aufgebaut ist und der Arm (14) mit dem Grundkörper (12) mechanisch, insbesondere formschlüssig, verbindbar ist,
das Spannen des Spannmechanismus (44, 48) selbsttätig mittels Federkraft (54) und das Lösen des Spannmechanismus (44, 48) mittels der Energiequelle (22) erfolgt und
ein Schieber (46) der Zylinder-Kolben-Mechanik (44) mittels der Energiequelle (22) in eine ausgefahrene Position bringbar ist, so dass bei Herstellung der mechanischen Verbindung zwischen Arm (14) und Grundkörper (12) gleichzeitig das Zugseil (48) mit dem Schieber (46) der Zylinder-Kolben-Mechanik (44) gekoppelt wird.

2. Chirurgische Vorrichtung (10) nach Anspruch 1, **dadurch gekennzeichnet, dass** die Energiequelle (22) extern, insbesondere außerhalb des Grundkörpers (12), ist.

3. Chirurgische Vorrichtung (10) nach Anspruch 1 oder 2, **dadurch gekennzeichnet, dass** die Energiequelle (22) über zumindest ein manuell betätigbares Betätigungselement (26, 28) ansteuerbar ist, das auf der distalen und/oder proximalen Seite des Arms (14) und/oder auf dem Grundkörper (12) angeordnet ist.

4. Chirurgische Vorrichtung (10) nach einem der Ansprüche 1 bis 3, **dadurch gekennzeichnet, dass**
die Energiequelle (22) eine hydraulische oder pneumatische Druckquelle oder eine elektrische Energiequelle ist;
die Zylinder-Kolben-Mechanik (44) hydraulisch, pneumatisch oder elektromotorisch betätigbar ist; und
über das zumindest eine Betätigungselement (26, 28) ein Fluidsteuerungsmittel zum Steuern des an der Zylinder-Kolben-Mechanik (44) wirkenden Fluiddrucks oder ein elektrisches Steuergerät zum Steuern der an einem Elektromotor wirkenden elektrischen Leistung zum Bewegen der Zylinder-Kolben-Mechanik (44) ansteuerbar ist.

5. Chirurgische Vorrichtung (10) nach einem der Ansprüche 1 bis 4, **dadurch gekennzeichnet, dass** die Gliederelemente (82, 82', 82") jeweils eine zentrale Zugseildurchführung (84) für das Zugseil (48) und zumindest eine außermittig angeordnete Aussparung (92) für Leitungen (90), insbesondere für eine Steuerleitung zwischen dem distalen Betätigungselement (26) und dem Fluidsteuerungsmittel oder dem elektrischen Steuergerät, aufweisen.

6. Chirurgische Vorrichtung (10) nach Anspruch 5, **dadurch gekennzeichnet, dass** die zentrale Zugseildurchführung (84) und die zumindest eine Aussparung (92) in einer im Gliederelement (82') drehbar gelagerten Drehscheibe (94) vorgesehen sind oder die Gliederelemente (82") eine Drehsicherung, insbesondere ineinander greifende Sperrelemente (96, 98), aufweisen, um eine Verdrehung der Gliederelemente (82") um das Zugseil (48) zu beschränken.

7. Chirurgische Vorrichtung (10) nach einem der Ansprüche 1 bis 6, **dadurch gekennzeichnet, dass** zwischen der zumindest einen Energiequelle (22) und dem Grundkörper (12) eine Adaptionseinheit (18) zwischengeschaltet ist, welche über lösbare Leitungen (20) einerseits mit dem Grundkörper (12) und andererseits mit der zumindest einen Energiequelle (22) verbunden ist.

8. Chirurgische Vorrichtung (10) nach Anspruch 7, **dadurch gekennzeichnet, dass** das Fluidsteuerungsmittel oder das elektrische Steuergerät in der Adaptionseinheit (18) angeordnet ist.

9. Chirurgische Vorrichtung (10) nach einem der Ansprüche 1 bis 8, **dadurch gekennzeichnet, dass** das Halteelement (16) mit einem universellen Adapter zum Anschließen verschiedenster einmal- oder wiederverwendbarer chirurgischer Instrumente und Geräte versehen ist.

10. Chirurgische Vorrichtung (10) nach einem der Ansprüche 1 bis 9, **gekennzeichnet, durch** einen chirurgischen Arbeitsarm (B) zum Stabilisieren oder Immobilisieren von bewegtem Gewebe oder zum Positionieren von Organen, insbesondere eines Teils eines schlagenden Herzens, mit
dem Gliederarm (14); und
dem zumindest einen Halteelement (16);
wobei der chirurgische Arbeitsarm (B) aufweist:
einen Kopplungsabschnitt (64) zur mechanischen und funktionalen Anbindung des Arbeitsarms (B) an der chirurgischen Vorrichtung (10), wobei das Zugseil (48) einen proximalen Verbindungsabschnitt (72) aufweist, der mit dem in der chirurgischen Vorrichtung (10) vorgesehenen Spannmechanismus (44) werkzeuglos, insbesondere formschlüssig, verbindbar ist.

11. Chirurgische Vorrichtung (10) nach Anspruch 10, **dadurch gekennzeichnet, dass** der Arbeitsarm (B) aufweist:
ein am distalen Ende des Gliederarms (14) vorgesehenes Betätigungselement (26) zur Ansteuerung des in der chirurgischen Vorrichtung vorgesehenen Spannmechanismus (44); und
Steuerleitungen (90), welche, insbesondere innerhalb des Gliederarms (14) verlaufend, das Betätigungselement (26) mit Schnittstellen (68) verbindet, die im Kopplungsabschnitt (64) zur Übertragung der Ansteuerungssignale oder -befehle zur chirurgischen Vorrichtung (10) vorgesehen sind.

## Claims

1. Surgical device (10) for stabilising or immobilising moving tissue, for example a part of a beating heart, or for positioning organs or for positioning and holding surgical instruments and apparatuses during a surgical intervention, comprising:
a base member (12) which can be fastened via a fastening portion (36) to a retaining device, in particular a sternum spreader, situated in or at an operation site;
a flexible arm (14), in particular an articulated arm, which can be fastened to the base member (12), which arm can be brought into different positions and/or states and at the free end of which at least one retaining element (16) is arranged;
a manually controllable power source (22); and
a tightening mechanism (44, 48) by which the arm can be fixed in a desired position and which has:
a pull rope (48) which is guided through a plurality of articulated elements (82; 82'; 82") of the arm (14) that are movable relative to one another and in particular formed in a complementary manner, and by which the articulated elements (82; 82'; 82") can be frictionally braced against each other; and
a cylinder-piston mechanism (44) which can be actuated via the power source (22) for actuating the pull rope (48),
**characterised in that**
the device (10) has a modular design and the arm (14) is mechanically, in particular positively, connectable to the base member (12),
the tightening mechanism (44, 48) is automatically tightened by means of spring force (54), and the tightening mechanism (44, 48) is released by means of the power source (22), and
a slider (46) of the cylinder-piston mechanism (44) can be brought into an extended position by means of the power source (22), so that, when the mechanical connection between the arm (14) and the base member (12) is made, at the same time the pull rope (48) is coupled to the slider (46) of the cylinder-piston mechanism (44).

2. Surgical device (10) according to claim 1, **characterised in that** the power source (22) is external, in particular outside the base member (12).

3. Surgical device (10) according to claim 1 or 2, **characterised in that** the power source (22) can be controlled via at least one actuating element (26, 28) which can be actuated manually and is arranged on the distal and/or proximal side of the arm (14) and/or on the base member (12).

4. Surgical device (10) according to any of claims 1 to 3, **characterised in that**
the power source (22) is a hydraulic or pneumatic pressure source or an electric power source;
the cylinder-piston mechanism (44) can be actuated hydraulically, pneumatically or by an electric motor; and
a fluid control means for controlling the fluid pressure acting on the cylinder-piston mechanism (44) or an electrical controller for controlling the electric power acting on an electric motor for moving the cylinder-piston mechanism (44) can be controlled via the at least one actuating element (26, 28).

5. Surgical device (10) according to any of claims 1 to 4, **characterised in that** each of the articulated elements (82, 82', 82") have a central pull rope passage (84) for the pull rope (48) and at least one eccentrically arranged recess (92) for lines (90), in particular for a control line between the distal actuating element (26) and the fluid control means or the electrical controller.

6. Surgical device (10) according to claim 5, **characterised in that** the central pull rope passage (84) and the at least one recess (92) are provided in a rotary disc (94) rotatably mounted in the articulated element (82') or the articulated elements (82") have an anti-rotation protection, in particular locking elements (96, 98) engaged in one another so as to restrict twisting of the articulated elements (82") around the pull rope (48).

7. Surgical device (10) according to any of claims 1 to 6, **characterised in that,** between the at least one power source (22) and the base member (12), an adapter unit (18) is interconnected which is connected via releasable lines (20) on one side to the base member (12) and on the other side to the at least one power source (22).

8. Surgical device (10) according to claim 7, **characterised in that** the fluid control means or the electrical controller is arranged in the adapter unit (18).

9. Surgical device (10) according to any of claims 1 to 8, **characterised in that** the retaining element (16) is provided with a universal adapter for connecting various single-use or re-usable surgical instruments and apparatuses.

10. Surgical device (10) according to any of claims 1 to 9, **characterised by** a surgical working arm (B) for stabilising or immobilising moving tissue or for positioning organs, for example a part of a beating heart, having
the articulated arm (14); and
the at least one retaining element (16);
wherein the surgical working arm (B) has:
a coupling portion (64) for mechanical and functional connection of the working arm (B) to the surgical device (10), wherein the pull rope (48) has a proximal connecting portion (72) which can be connected without using tools, in particular positively, to the tightening mechanism (44) provided in the surgical device (10).

11. Surgical device (10) according to claim 10, **characterised in that** the working arm (B) has:
an actuating element (26) provided at the distal end of the articulated arm (14) for controlling the tightening mechanism (44) provided in the surgical device; and
control lines (90), in particular extending inside the articulated arm (14), which connect the actuating element (26) to interfaces (68) provided in the coupling portion (64) for transmission of the control signals or instructions to the surgical device (10).

## Revendications

1. Dispositif chirurgical (10) destiné à stabiliser ou à immobiliser un tissu déplacé, par exemple une partie d'un coeur battant, ou à positionner des organes ou à positionner et à maintenir des instruments chirurgicaux et des appareils pendant une intervention chirurgicale, avec :
un corps de base (12), qui peut être fixé par l'intermédiaire d'une partie de fixation (36) à un dispositif de maintien situé dans ou sur le champ opératoire, en particulier un écarteur sternal ;
un bras flexible (14) pouvant être fixé au corps de base (12), en particulier bras articulé, qui peut être amené dans différentes positions et/ou situations et à l'extrémité libre duquel est disposé au moins un élément de maintien (16) ;
une source d'énergie (22) pouvant être commandée manuellement ; et
un mécanisme de serrage (44, 48), par l'intermédiaire duquel le bras peut être bloqué dans une position souhaitée, et lequel présente :
un câble de traction (48), qui est guidé par une pluralité d'éléments articulés (82 ; 82' ; 82") du bras (14) mobiles les uns par rapport aux autres, en particulier réalisés de manière complémentaire, et par l'intermédiaire duquel les éléments articulés (82 ; 82' ; 82") peuvent être serrés les uns contre les autres par friction ; et
un mécanisme piston-cylindre (44), pouvant être actionné par l'intermédiaire de la source d'énergie (22), destiné à actionner le câble de traction (48),
**caractérisé en ce que**
le dispositif (10) est à structure modulaire et le bras (14) peut être relié au corps de base (12) de façon mécanique, en particulier par coopération de formes,
le serrage du mécanisme de serrage (44, 48) s'effectue automatiquement au moyen d'une force élastique (54) et la libération du mécanisme de serrage (44, 48) s'effectue au moyen de la source d'énergie (22) et
un tiroir (46) du mécanisme piston-cylindre (44) peut être amené au moyen de la source d'énergie (22) dans une position sortie, de sorte que, lors de l'obtention de la liaison mécanique entre le bras (14) et le corps de base (12), le câble de traction (48) est accouplé simultanément au tiroir (46) du mécanisme piston-cylindre (44).

2. Dispositif chirurgical (10) selon la revendication 1, **caractérisé en ce que** la source d'énergie (22) est externe, en particulier à l'extérieur du corps de base (12).

3. Dispositif chirurgical (10) selon la revendication 1 ou 2, **caractérisé en ce que** la source d'énergie (22) peut être commandée par l'intermédiaire d'au moins un élément d'actionnement (26, 28) pouvant être actionné manuellement, qui est disposé sur la face distale et/ou proximale du bras (14) et/ou sur le corps de base (12).

4. Dispositif chirurgical (10) selon l'une quelconque des revendications 1 à 3, **caractérisé en ce que**
la source d'énergie (22) est une source de pression hydraulique ou pneumatique ou une source d'énergie électrique ;
le mécanisme piston-cylindre (44) peut être actionné de manière hydraulique, pneumatique ou électromotrice ; et
un moyen de commande de fluide destiné à commander la pression de fluide agissant sur le mécanisme piston-cylindre (44) ou un appareil de commande électrique destiné à commander la puissance électrique agissant sur un moteur électrique pour déplacer le mécanisme piston-cylindre (44) peut être commandé par l'intermédiaire de l'au moins un élément d'actionnement (26, 28).

5. Dispositif chirurgical (10) selon l'une quelconque des revendications 1 à 4, **caractérisé en ce que** les éléments articulés (82, 82', 82") présentent respectivement un passage de câble de traction (84) central pour le câble de traction (48) et au moins un évidement (92) disposé de manière excentrée pour des lignes (90), en particulier pour une ligne de commande entre l'élément d'actionnement (26) distal et le moyen de commande de fluide ou l'appareil de commande électrique.

6. Dispositif chirurgical (10) selon la revendication 5, **caractérisé en ce que** le passage de câble de traction (84) central et l'au moins un évidement (92) sont prévus dans un disque tournant (94) monté en rotation dans l'élément articulé (82') ou les éléments articulés (82") présentent une sécurité anti-rotation, en particulier des éléments de blocage (96, 98) s'interpénétrant, afin de limiter une torsion des éléments articulés (82") autour du câble de traction (48).

7. Dispositif chirurgical (10) selon l'une quelconque des revendications 1 à 6, **caractérisé en ce qu'une** unité d'adaptation (18), laquelle est reliée d'une part au corps de base (12) et d'autre part à l'au moins une source d'énergie (22) par l'intermédiaire de lignes (20) détachables, est intercalée entre l'au moins une source d'énergie (22) et le corps de base (12).

8. Dispositif chirurgical (10) selon la revendication 7, **caractérisé en ce que** le moyen de commande de fluide ou l'appareil de commande électrique est disposé dans l'unité d'adaptation (18).

9. Dispositif chirurgical (10) selon l'une quelconque des revendications 1 à 8, **caractérisé en ce que** l'élément de maintien (16) est pourvu d'un adaptateur universel destiné à raccorder des instruments et appareils chirurgicaux à usage unique ou réutilisables les plus divers.

10. Dispositif chirurgical (10) selon l'une quelconque des revendications 1 à 9, **caractérisé par** un bras de travail chirurgical (B) destiné à stabiliser ou à immobiliser un tissu déplacé ou à positionner des organes, en particulier une partie d'un coeur battant, avec
le bras articulé (14) ; et
l'au moins un élément de maintien (16) ;
dans lequel le bras de travail chirurgical (B) présente :
une partie d'accouplement (64) destinée au rattachement mécanique et fonctionnel du bras de travail (B) au dispositif chirurgical (10), dans lequel le câble de traction (48) présente une partie de liaison proximale (72), qui peut être reliée sans outil, en particulier par coopération de formes, au mécanisme de serrage (44) prévu dans le dispositif chirurgical (10).

11. Dispositif chirurgical (10) selon la revendication 10, **caractérisé en ce que** le bras de travail (B) présente :
un élément d'actionnement (26) prévu sur l'extrémité distale du bras articulé (14) pour commander le mécanisme de serrage (44) prévu dans le dispositif chirurgical ; et
des lignes de commande (90), que, en s'étendant en particulier à l'intérieur du bras articulé (14), l'élément d'actionnement (26) relie à des interfaces (68), qui sont prévues dans la partie d'accouplement (64) pour la transmission des signaux ou instructions de commande au dispositif chirurgical (10).
